# EUROPEAN PATENT APPLICATION

(11) **EP 4 202 030 A1**
(43) Date of publication of application: **28.06.2023**
(21) Application number: 21216595.5
(22) Date of filing: 21.12.2021
(51) Int. Cl.: C12M 1/12, C12M 1/00, C12M 3/00, A61J 1/14

(54) **A TANK FOR A BIO-PHARMA PROCESS**

(71) Applicant: ESTR Biosystems GmbH, 37083 Göttingen (DE)
(72) Inventor: Schlack, Stefan, 37083 Göttingen (DE)
(74) Representative: Bardehle Pagenberg Partnerschaft mbB Patentanwälte Rechtsanwälte

(57) **Abstract**

The present invention relates to a reservoir element 200 for a tank 1 of a bio-pharma process line, the reservoir element 200 including a body element 210, and at least one shell element 220, 222. The body element 210 has a first end 212 and a second end 214, being opposite the first end, each of the first and second ends including an orifice, and wherein the body element tapers from the first end to the second end, wherein an inner volume is defined between the first and second ends. The body element and/or the at least one shell element include at least one groove 216, 217, wherein the at least one shell element 220, 222 is fixedly attached to the body element 210 so that the body element and the at least one shell element sandwich the groove in between forming at least one channel, the at least one channel being adapted for guiding at least one biochemical medium and/or an operating medium.

## Description

### Field of the Invention

The present invention relates to a reservoir element for a tank, a tank assembly, a tank, a tank system including a clean room bag and a method for assembling a tank. The tank and/or the tank system may be used in a bio-pharma process, and may further be adapted for manufacturing and/or developing e.g. bio-pharmaceutical goods and/or for developing and/or testing the commercial manufacturing process of the same.

### Background Art

Bio-pharmaceutical goods, such as drugs, e.g. for cancer therapy, genetic therapy and/or cell therapy, are nowadays manufactured in so called bio-pharma process lines.

Known bio-pharma process lines are e.g. based stainless-steel tanks that are interconnected by pipes and/or the like. Those known bio-pharma process lines are difficult to maintain and to clean. Thus, so called single-use bio-pharma process lines were developed that are replaced after usage. Here, single-use containers, such as bags, serve as reservoirs for educts and/or products of the bio-pharma process line. Said bags are typically supported in rigid containers, such as stain-less steel containers, and are interconnected by hoses. These known rigid containers are bulky and require lots of storage space, when being not in use, leading to increased storing and transport costs.

For setting up a conventional single-use bio-pharma process line a complex manual assembly is required, leading to increased costs. For example, multiple bags, filters, sensors, valves, etc., have to be connected via various hoses. The complex assembly bears a significant risk for mal-function of the bio-pharma process line, due to improper or incorrect assembling.

Further, the single-use bio-pharma process lines are prone to damages as the (hose-based) connections and/or bags of the single use containers tend to leakages over the operational time. In case of leakage or mal-function, the educts and/or products of the entire bio-pharma process line can be damaged or even destroyed. Accordingly, there is a significant economic risk for the operator of the bio-pharma process line.

Still further, known single-use bio-pharma process lines typically include different materials that are in contact with the educts and/or products, thereby increasing the risk of contamination of the same. This contamination may be caused by extractables and particularly leachables initially contained in the different materials of the bio-pharma process line. Further, cell growth and/or other bio-chemical process steps may be distorted. Particularly, if pumps are used to transport the educt- and/or product-containing fluids through the bio-pharma process line, there is a risk of particle generation, due to abrasion.

Due to the complex built of the single-use bio-pharma process lines, automatization of assembling and operating the bio-pharma process lines is very difficult and expensive. Thus, in particular smaller bio-pharma process lines are oftentimes manually operated.

Further, after usage, all components of the single-use bio-pharma process line are typically discarded, as cleaning or recycling is oftentimes impossible.

In view of the above, it is an object of the present invention to provide a reservoir element for a tank, a tank and a tank system that may be used in a bio-pharma process line as well as a tank assembly and a method for assembling a tank, that overcome the above-mentioned drawbacks. Further, costs and time required for developing and manufacturing bio-pharmaceutical goods shall be reduced, thereby allowing a faster time-to-market. Particularly, the tank and/or the tank system shall be adapted to be operated automatically. Further the tank and/or the tank system shall be adapted to be assembled automatically.

### Summary of the Invention

The object is achieved by a reservoir element, a tank, a tank assembly, a tank system, a clean room bag and a method for assembling a tank according to the independent claims. Further embodiments are described in the dependent claims and the following description. Parts of the description that do not fall under the scope of the claims are provided to give a better understanding of the claimed invention.

In particular, the object is achieved by a reservoir element for a tank of a bio-pharma process line, the reservoir element includes a body element and at least one shell element. The body element has a first end and a second end, being opposite the first end, each of the first and second ends including an orifice, wherein the body element tapers from the first end to the second end. Further, an inner volume is defined between the first and second ends.

The body element and/or the at least one shell element include at least one groove, wherein the at least one shell element is fixedly attached to the body element so that the body element and the at least one shell element sandwich the groove in between forming at least one channel. The at least one channel is adapted for guiding at least one biochemical medium and/or an operating medium.

The body element may be made by injection moulding, injection blow moulding, extrusion blow molding or thermoforming. Hence, lateral walls of the inner volume are integrally formed, thereby increasing the tightness of the reservoir element and particularly of a tank including said reservoir element. Further, injection molded, or thermoformed elements can be manufactured cost efficient and with high quality. Thus, the costs for a tank and/or a tank system for a bio-pharma process line can be reduced, compared to conventional bio-pharma process lines.

The at least one shell element may be fixedly attached to a single lateral wall or multiple lateral walls of the body element. For example, the shell element may be formed to cover all lateral walls of the body element, when being attached. In an other embodiment, the shell element may be formed to cover only one, or some (at least two) lateral walls of the body element, when being attached. The shell element may be attached on the inside or the outside of the body element.

Particularly, the at least one shell element may be welded to the body element, preferably by ultrasonic welding, or other suitable welding techniques (laser welding, clear to clear welding, friction welding). In a further embodiment, the at least one shell element is glued to the body element. For example, reactive or non-reactive adhesives, such as hot melt adhesives can be used. Additionally, or alternatively, the shell element(s) may be attached to the body element using mechanical fasteners, such as screws, bolts, rivets, and/or the like. For sealing the body element and the shell element, additionally, a seal member may be provided. The seal member may be a sealing gasket or a liquid sealing.

The body element and the shell element may formed from a plastic material, preferably from the same plastic material. For example, the body element and the shell element may be formed from COC (cyclic olefin copolymer), COP (cyclic olefin polymer), PP (polypropylene), PC (poly carbonate), PET (polyethylene terephthalate), and/or the like The groove that is included in at least one of the body element and/or the at least one shell element may have any suitable cross-section (e.g. semi-circular, triangular, quadrangular, or the like). Particularly, a first groove may be formed in the body element and a second corresponding groove may be formed in the shell element. Thus, when attaching the shell element to the body element, a channel is formed by the first and second grooves. For example, a channel with circular cross-section can be formed by a semi-circular first groove and a corresponding semi-circular second groove.

Forming channels using groves allows to form channels with complex geometries that cannot be formed by injection moulding an integral part. For example, a heating or cooling channel can include several windings within a lateral wall of the reservoir element.

The at least one channel may be chosen from a group of different channel-types, comprising the following channel types: an inlet channel, an outlet channel, a bypass channel, a heating or cooling channel, a sampling channel, or the like.

An inlet channel serves for guiding a biochemical medium and/or an operating medium to the inner volume (i.e. a reservoir of an assembled tank). The inlet channel may comprise a sparger or may be coupled to a sparger. An outlet channel serves for removing a biochemical medium and/or an operating medium from the inner volume (i.e. a reservoir of an assembled tank). When being connected to a further (second) tank, the outlet channel of a first tank may be connected to an inlet cannel of the second tank, so as to transfer medium from the reservoir of the first tank to a reservoir of the second tank.

Further, a channel may be provided that serves for transferring a retentate from e.g. a filter or a membrane back into the inner volume, respectively a tank. This channel may be a separate retentate channel or said channel may be integrally formed with an outlet channel. For transferring a retentate back, a filter or membrane can be flown through with an operating fluid, in a direction opposite to the operating direction. The operating direction is the direction in which the medium flows for filtering. During filtering, permeate goes through the membrane/filter, retentate is hold back by the membrane/filter.

A further channel may be provided that serves for transferring permeate and/or filtrate back into the inner volume, respectively a reservoir of a tank and/or to another tank. For transferring a permeate and/or filtrate a positive pressure may be applied on a permeate side, or a filtrate side of a filter, respectively. This may be achieved by guiding an operating medium, such as pressurized (sterile) air, to the permeate side or filtrate side, respectively. The operating medium urges the permeate towards a permeate channel that may then guide the permeate/filtrate back into the reservoir of the assembled tank and/or to another tank. The direction of flow can be controlled by opening/closing respective valves that can be associated with the respective channels.

Further channels may be provided that serve for recirculating a medium in an assembled tank (recirculation channel); wetting or flushing components of the assembled tank, particularly at least one filter (wetting channel, flushing fluid channel); for removing products (product channel);for providing medium (feed channel); for removing/recirculating permeate/filtrate (permeate or filtrate channel); for removing waste (waste channel); for harvesting cells (cell bleed channel); for suppling, removing and/or transferring cells (cell channel); for pressurizing at least portions of the assembled tank (pressure channel) and/or for loading different solutions to the assembled tank, particularly to cartridges for chromatography, i.e. for washing, cleaning, eluding (washing channel, cleaning channel, eluding channel).

A bypass-channel serves for guiding a biochemical medium and/or an operating medium, wherein the bypass-channel is not connected to the inner volume of the reservoir element (respectively not to the reservoir of the assembled tank). Thus, a medium can bypass the assembled tank without being in communication with the reservoir of the assembled tank. For example, when three tanks are connected so that a first and a third tank sandwich a second tank, a fluid may be guided from the reservoir of the first tank to a reservoir of the third tank without passing a reservoir of the second tank. In this case, the bypass-channel may be provided in a reservoir element of the second tank. Optionally, the bypass-channel may be adapted to be fluidically separated from or connected to the inner volume or a respective reservoir of an assembled tank. This can be achieved e.g. by a valve. Depending on the valve position (open/closed), the bypass-channel may be or may not be in communication with the inner volume / the reservoir of the tank. Thus, in a process line, the flow of the medium can be controlled in that the medium bypasses the reservoir of the tank, or not.

For example, the bypass-channel allows to provide a biochemical medium, such as a buffer, that is used in various tanks of a bio-pharma process line in a large storage tank. Said biochemical medium can then be transferred from the storage tank to all tanks that require said medium (e.g. buffer). In case a tank does not require said medium, the medium can bypass the reservoir of said tank. Further, by providing a valve in the bypass channel, the supply of said medium can be controlled (e.g. amount and time). Further, the bypass-channel may serve for transferring a bio-chemical medium, such as a cell culture, from a first tank (e.g. a seed tank) in at least two subsequent tanks that may serve as further bioreactors. Thus, a bio-chemical medium can be guided from a single tank to multiple subsequent tanks.

A heating or cooling channel serves for guiding a tempered heating or cooling medium, for tempering an assembled tank. Depending on the degree of heating/cooling, the medium received in the tank may evaporated or condensate. Further, the reservoir element may comprise at least one longitudinal rib. The at least one longitudinal rib may protrude into the inner volume. The heating or cooling channel may be at least partially received within the at least one longitudinal rib. Thus, the efficiency of heating or cooling may be increased.

A sampling channel serves for taking a sample of biochemical medium and/or of operating medium from the reservoir of the assembled tank. Thus, educts, products and intermediate products or the process line can be removed and analyzed.

Particularly, the reservoir element may comprise multiple channels formed by respective grooves formed in the body element and/or the at least one shell element. Those channels may be adapted to guide different biochemical mediums and/or operating mediums if the reservoir element is assembled in a tank and the tank is in use. By providing respective reservoir elements, the functionality of an assembled tank can be adapted to the specific needs of the respective process line.

In particular, an assembled tank may be a multi-functional tank comprising multiple channels of different channel-types and/or the same channel type. At least one channel maybe opened and closed, using e.g. a valve, a closure, or the like. Thus, depending on desired functionality a channel or multiple channels can be closed. These closed channels are not used. At least one other or multiple other channels may be opened and therefore used, thereby defining the functionality of the multi-functional tank.

At least one of the multiple chancels may be in communication with the inner volume (i.e. a part of a reservoir of an assembled tank) and/or may bypass the inner volume. Further, the reservoir element may include an additional channel formed in a tube, such as a rigid tube, that can be inserted in the inner volume, e.g. through an opening in the body element and/or a plate element. Said tube may be sealingly received in said respective opening. The additional channel may be configured for guiding at least one biochemical medium and/or an operating medium from the outside of an assembled tank into the reservoir of said tank.

The channel(s) may have an equivalent diameter in the range of 0.1 to 3 inch (0.25 to 7.6 cm), preferably in the range of 0.2 to 2 inch (0.5 to 5.1 cm), more preferably in the range of 0.3 to 1.5 inch (0.75 to 3.8 cm), even more preferably in the range of 0.5 to 1 inch (1.2 to 2.5 cm) and most preferably about 0.75 inch (1.9 cm). Further, different channels may have different diameters and/or the diameter of the channel may vary. Thus, e.g. nozzles maybe provided. The equivalent diameter of a channel having a non-circular cross section is equal to a diameter of a channel having a circular cross section, wherein both channels (non-circular and circular) have the same cross-sectional area. For channels with a circular cross section, hence, the diameter and equivalent diameter are the same.

Further, the body element may have a polygonal cross-section in a plane parallel to the first end, wherein the at least one shell element may be plate shaped. A polygonal cross section allows to provide substantially flat lateral walls. Said substantially flat lateral walls facilitated attaching the shell element(s) to the respective lateral wall of the body element. Alternatively, the shell element may also have a polygonal cross-section. In a further alternative, the shell element may be formed to cover only some, for example at least two, lateral walls of the body element when being attached.

The inner volume and the first orifice may be configured so that the reservoir element is adapted to receive a further reservoir element at least partially, so that multiple reservoir elements are stackable within each other. Hence, the reservoir elements can be easily transported and stored, requiring little storage space. Thus, costs can be further reduced.

Further, the reservoir element may comprise a plate element. The plate element being sealingly attached to the first or second end of the body element, so as to cover the respective orifice. The plate element may include at least one channel, wherein the at least one channel is in communication with the channel formed by the body element and the at least one shell element.

The plate element may be sealingly attached to the first or second end of the body element for example by welding, preferably by ultrasonic welding, or other suitable welding techniques (e.g. laser welding, clear to clear welding, friction welding). In a further embodiment, the at least one shell element is glued to the body element. For example, reactive or non-reactive adhesives, such as hot melt adhesives can be used. Additionally, or alternatively, the plate element may be attached to the body element using mechanical fasteners, such as screws, bolts, rivets, and/or the like. For sealing the body element and the plate element, additionally, a seal member may be provided. The seal member may be a sealing gasket or a liquid sealing. The plate element may be an integrally formed part or may be formed from multiple layers. For example, the plate element may have a two-layer structure, wherein a first and /or second layer include at least one groove, wherein the first layer is fixedly attached to the second layer that the first and second layers sandwich the groove in between forming at least one channel. The at least one channel is adapted for guiding at least one biochemical medium and/or an operating medium. Further, the channel may be in fluid communication with a channel formed between the body element and the at least one shell element.

The reservoir element may also include at least one insert, adapted to cover an undercut of the inner volume at least partially. The insert may be integrally formed with the body element and/or the plate element or may be a separate part. The insert allows to cover an undercut of the inner volume, so that dead spaces within the inner volume can be prevented or at least reduced. Further, the insert may enhance the mechanical stability of the reservoir element.

In an alternative embodiment, the reservoir element maybe formed using a 3D-printing technique. For example, the body element and the at least one shell element can be integrally formed. In this embodiment, the grooves/channels may be integrally formed within a wall of the reservoir element. Further, the plate element and/or the insert may also be formed using a 3D-printing technique. Thus, the entire reservoir element can be integrally formed.

The first end and/or second end of the reservoir element may include a sealing face, for sealingly connect the reservoir element to a further reservoir element. Particularly, the sealing face may include at least one sealing member for providing a sealed connection between adjacent reservoir elements. The sealing member maybe arranged circumferentially around the orifice of the first and/or second end. When the reservoir elements are assembled to form a reservoir of a tank, the sealing member may be compressed.

Further, the reservoir element and particularly the plate element may include at least one port, wherein the at least one port is associated with a respective channel, and wherein the port is chosen from a group of port-types, comprising the following port-types:
∘ a fluid inlet port;
∘ a gas inlet port;
∘ a fluid outlet port;
∘ a heating/cooling port;
∘ a gas outlet port;
∘ a cell bleed port,
∘ a cell transfer port,
∘ a medium supply port,
∘ a medium remove port,
∘ an element-interconnecting port, and
∘ a tank-interconnecting port.

The (fluid or gas) inlet port serves for providing a biochemical medium and/or an operating medium to an inner volume. In an assembled state, the inner volume may form part of a reservoir of a tank of a bio-pharma process line. A heating/cooling port serves for proving a tempered heating/cooling fluid to a heating/cooling channel. A (fluid or gas) outlet port serves for removing a biochemical medium and/or an operating medium from the inner volume or a respective reservoir of a tank. A medium supply port allows to supply medium (biochemical medium and/or an operating medium) to the inner volume/tank and a medium remove port allows to remove medium (or parts thereof) from the inner volume/tank. The medium remove port may also serve for transferring a retentate from e.g. a filter or a membrane back into the inner volume/tank. A tank-interconnecting port serves for coupling a channel of a reservoir element of a first tank fluidically to a corresponding channel of a reservoir element of a second tank, when the first tank is connected with the second tank, e.g. by using a connector means as will be described in greater detail below. The tanks may be connected side by side (first tank adjacent to second tank), or the first tank maybe provided on top of the second tank. A tank-interconnecting port may serve as medium supply port and/or medium remove port. A cell bleed port serves for removing cells from the inner volume/reservoir of a tank. The cells may either be discarded or may be transferred to a further tank or other device for further processing.

The at least one port may be associated with a valve that can preferably be controlled by a handling manipulator (or multiple handling manipulators). Thus, the flow of medium from and to the tank can be controlled by opening/closing the valve. Particularly, the valve may be a flow control valve, that allows to adjust the flow gradually.

For example, a channel may be associated with a tank-interconnecting port and a fluid outlet port. Thus, said channel may serve for transferring a medium, such as a fluid, from the inner volume/reservoir of the tank to a further tank. In case a valve is associated with the tank-interconnecting port, the flow of the transferred medium can be controlled. Alternatively, or additionally, the flow may be controlled by controlling the pressure in the associated tanks (positive and/or negative pressure).

The at least one port may comprise a protruding shroud that at least partially surrounds the associated channel. The shroud may be concentrically arranged around a channel end of the associated channel. Further, the at least one port may comprise a recess, that at least partially surrounds the associated channel. The recess may be concentrically arranged around a channel end of the associated channel. Said port may be adapted to be coupled to a hose element or a pipe element so as fluidically connect the reservoir element and/or the tank with further objects in the periphery. For example, a tank comprising said reservoir element may be integrated in a conventional bio-pharma process line. For connecting channels/tanks, the port may include a snap fit connection member, that is adapted to engage with a corresponding snap fit connection member. This snap fit connection may provide a releasable and tight connection.

Further, the port (e.g. a tank-interconnecting port or an element-interconnecting port) maybe adapted to engage with a corresponding port (e.g. a corresponding tank-interconnecting port or an element-interconnecting port), wherein the port and the corresponding port are formed to provide a positive locking. For example, the reservoir element may comprise a port on a first side and a corresponding port on a second side, wherein the first side and the second side may be opposing outer surfaces of the reservoir element. A tank including said reservoir element can be coupled with a further tank, using the port and the corresponding port and to fluidically connect the respective channels when coupling the tanks. Thus, a medium can be transferred from a first tank to a second tank without the use of intermediate conduit elements, such as pipes or hoses. Thereby assembling a tank system of e.g. a bio-pharma process line is facilitated and less prone to assembly mistakes.

Further, a first reservoir element of a tank may comprise a port and a second reservoir element of the tank may comprise a corresponding port. This allows to couple the reservoir elements to form a reservoir of the tank and thereby to fluidically connect the respective channels of the reservoir elements when assembling the tank. Thus, a medium can be guided though a channel formed in at least two reservoir elements of the tank. Thereby assembling a tank is facilitated and less prone to assembly mistakes.

The ports may include a sealing member (e.g. flexible sealing member, such as a rubber seal, a silicon seal, a Teflon seal, or the like). Said sealing member may be a radial and/or an axial sealing. For example, the sealing member may be provided on a shroud and/or within a recess of the port. The sealing member maybe provided as sealing gasket that allows to seal multiple ports of the reservoir element. Further, the sealing member may be port specific and adapted to seal only a single port.

The reservoir element may also comprise multiple tank-interconnecting ports to provide an interconnecting interface that allows to easily couple a tank to a further tank. Further, each reservoir element of a tank may comprise multiple element-interconnecting ports to provide an interconnecting interface that allows to easily couple different reservoir elements of the tank to assemble the tank.

Still further, the reservoir element and particularly the plate element may include at least one filter, wherein the at least one port may be covered by the at least one filter, and wherein the filter may be chosen from a group of filter-types, comprising the following filter-types:
∘ a pre-filter;
∘ a sterile filter;
∘ a bacterial filter;
∘ a viral filter;
∘ a mycoplasma filter;
∘ an ultrafiltration filter;
∘ a diafiltration filter;
∘ a cell filter;
∘ a cell harvest filter;
∘ a fluid filter;
∘ a bio-burden filter
∘ an air filter, and
∘ a gas filter.

The filter may be provided within the inner volume of the reservoir element, thus, when being assembled to a tank, the filter may be provided in the reservoir of the tank. Thus, the respective tank may serve as filtration unit for filtering the medium received in the reservoir of the tank. The air filter may be a syringe filter. The filter may be a liquid filter. The liquid filter may serve to provide a buffer for flushing.

Further, the filter may be integrated into a reservoir element, particularly into a shell element or a plate element of the respective reservoir element. For example, a reservoir element (e.g. a top plate element of a tank or a sidewall element) maybe integrally formed with a filter housing.

Further, at least one port of the reservoir element may be covered by a filter. Providing a filter-covered port allows to withhold parts of the medium in the assembled tank and/or prevent other parts of a medium form entering the tank. In case an inlet port is covered with a filter, only filtrate can enter the inner volume of the reservoir element, respectively the tank. In case an outlet port is covered, only the filtrate is allowed to leave the inner volume of the reservoir element, respectively the tank. For example, a gas inlet port may be covered with a sterile filter. Thus, sterile pressurized air can be guided into the inner volume of the reservoir element, respectively the tank and thus, medium contained in the reservoir of the tank can be blown out of the reservoir, e.g. for being transferred to a further tank.

Further, the filter covering the at least one port may be heated and/or cooled. Thus, medium entering and/or leaving the reservoir element (respectively the tank) can be tempered to a desired temperature. Further, heating the filter allows to provide a hot filtration and cooling the filter allows to provide a cold filtration. Heating a filter further serves to prevent undesired condensation.

Further, the reservoir element and particularly the plate element may include at least one valve, the at least one valve being associated with the at least one channel, wherein the valve may be a flow control valve, a cutoff valve, a pressure relief valve or a non-return valve, or the like. Further, the reservoir element may comprise multiple valves of the same and/or of different types. Particularly, a valve may be provided at a junction of at least two channels, being formed in the reservoir element. Thus, depending on the valve position, medium can be guided to different channels and/or ports. Hence, the fluid flow can be controlled.

A flow control valve allows to control the amount of medium flowing through the channel per unit of time (e.g. flow in liters/second). The flow control valve may be configured to control the flow dynamically, to control a biochemical process in a tank, including said reservoir element. A cutoff valve allows to open or close the channel. A cut off valve may be provided in a bypass-channel. Thus, the bypass-channel may be fluidically separated from the inner volume or a respective reservoir of the tank (valve closed) or in communication with the reservoir of the inner volume/tank (valve open). Further a cutoff valve allows to close channels that are not used and open channels that are used in the tank, depending on the functionality of the tank.

A non-return valve may be provided in a channel to prevent medium that shall be removed from the inner volume/the tank to flow back into the inner volume/ the tank. Thus, e.g. contamination of the inner volume/ the tank can be prevented. Further, a non-return valve may prevent medium that has entered the inner volume / the tank from flowing back to its origin. Thus, a tank can be pressurized (e.g. by pressurized air) and medium contained in the reservoir of the tank can be guided in a desired direction, for example for filtration purposes. Using pressurized tanks allows to guide the medium without using pumps. Thus, contamination of the medium and particle generation (e.g. due to abrasion) can effectively be reduced or even prevented. Further, no (expensive) pumps, such as single use pumps or pump heads, are needed, thereby reducing the costs of the tank and/or the tank system for a bio-pharma process line. Still further, avoiding pumps facilitates cleaning, maintaining and/or sterilizing the tank.

The at least one valve can have any suitable configuration. For example, the at least one valve can be a ball valve, a butterfly valve, a diaphragm valve, a gate valve, a needle valve, a pinch valve, or the like.

The at least one valve may be configured to be actuated manually and/or automatically. For example, the valve can be configured to be actuated mechanically, pneumatically, hydraulically, magnetically, electrically, and/or the like. Further, the at least one valve may be configured to be actuated from the outside of the tank, by means of an actuating means. In particular, the valve may be a mechanical valve that is configured to be actuatable from the outside of the tank, by means of an actuating means. The actuating means may be an actuating rod, that connects a valve closure member that is in contact with the medium with the outside of the tank. The actuating rod maybe supported and sealed in at least one reservoir element forming the tank. Further, the actuating rod may include several sections, wherein each section of the actuating rod is supported in a respective reservoir element. When assembling a tank from multiple reservoir elements, the sections of the actuating rod can be coupled, so as to allow to actuate a valve in a first reservoir element, by initially actuating an actuating rod of a further (e.g. second) reservoir element.

For opening/closing the valve, the actuating means can be rotated or axially displaced, depending on the type of associated valve. Further, the actuating means may include a magnet (permanent or electro-magnet), that is adapted to open/close the valve. The magnet allows to actuate a respective magnetic valve from the outside of the tank. Providing a magnetic actuating means facilitates the sealing of the valve, as the valve closure member and the actuating means can be separated from each other, e.g. by a continuous wall portion.

In a particular embodiment, the valve is closed in the initial state and can be opened by axially displacing a valve body. This can e.g. be done by an actuating rod or by using a magnetic force. In the closed initial state, the valve may be pre-loaded by means of a spring member or a magnetic member. Hence, when being not actuated, the valve may be in a closed state. Only when being operated (e.g. manually or via a handling manipulator) the valve opens.

The actuating means may be adapted to couple with an actuating mechanism, that may be part of a handling manipulator that allows automated control of the actuating means and thus of the tank and/or a tank system.

Particularly, when using mechanical and/or magnetically valves it is possible to avoid electric valve components being integrated in the tank. Thus, recycling of the tank is facilitated and improved.

The valve, valve body and/or actuating means may be initially integrated in the respective reservoir element of the tank or may be adapted to be integrated in the respective reservoir element of the tank after or during assembly of the tank. This facilitates the assembly of the tank and/or a tank system of a bio-pharma process lines. This is, as in conventional bio-pharma process lines valves are typically provided as separate (single-use) components that have to be connected manually to other part of the process line, such as bags, and/or the like.

Further, an inner surface of the inner volume and/or the at least one channel may be coated. Further, at least the components of the valves, ports and/or filters of the reservoir element that can come into contact with a biochemical and/or operating medium may be coated. The coating may be a silicon dioxide coating, a glass-based coating and/or the like. The coating reduces the number of different contact materials that are in contact with the biochemical and/or operating medium. Thus, the risk of contamination of the biochemical and/or operating medium and/or the risk of distortion of cell growth and/or other bio-chemical process steps can be reduced.

Further, the coating may form a gas barrier and/or may provide an inert inner surface of the inner volume and/or the at least one channel. The coating may be temperature resistant to allow heating and/or cooling the reservoir element and a respective tank. Further, recycling of the reservoir elements can be facilitated, as the reservoir elements forming a tank can be formed of the same (partially coated) materials. The coating can be applied during or after manufacturing of the reservoir elements, i.e. e.g. during injection molding and/or thermoforming.

The reservoir element may further comprise at least one assembly-connecting means and/or at least one corresponding assembly-connecting means, wherein the assembly-connecting means and the corresponding assembly-connecting means are configured to engage with each other, so as to secure an assembly of at least two adjacent reservoir elements, wherein the engagement of the assembly-connecting means and the corresponding assembly-connecting means. Those assembly-connecting means may be configured to be engaged with a corresponding connecting-means automatically, e.g. using an assembling manipulator.

For example, the assembly-connecting means may be a threaded member that is integrated into the reservoir element. The threaded member may have an internal thread or an external thread and may be integrally formed with the reservoir element. Optionally or additionally, the threaded member may be an inlay, such as a metal inlay, that is securely held in the reservoir element. An inlay can for example be overmolded or glued into the reservoir element. The corresponding assembly-connecting means can be a trough opening that can be aligned with the threaded member having an inner thread. Thus, reservoir elements can be connected and engaged by threading a screw through the through opening into the threaded member. Further, the corresponding assembly-connecting means can be a trough opening that receives a threaded member having an outer thread. Thus, the reservoir elements can be connected and engaged by threading a nut and or the like on the threaded member, thereby engaging the reservoir elements.

Further, the assembly-connecting means may be protrusions, such as bolts. The protrusions can be integrally formed with the reservoir element or can be an inlay. The inlay can for example be overmolded or glued into the reservoir element. The corresponding assembly-connecting means may be a corresponding recess. The recess may be integrally formed with the reservoir element or can be an inlay, such as a sleeve. The inlay can for example be overmolded or glued into the reservoir element. During assembly, the assembly-connecting means engage with the corresponding assembly-connecting means and provide a positive locking. The engagement of the assembly-connecting means and the corresponding assembly-connecting means may be a self-retaining engagement. The self-retaining engagement may be achieved by a retaining force provided by a flexible member that may be provided between reservoir elements that are engaged (e.g. a top plate element and a sidewall element, two sidewall elements and/or a bottom plate element and a sidewall element). Due to assembly, the flexible member may be compressed, thereby providing a retaining force. Particularly, the flexible member may be a sealing member that seals the reservoir formed by the reservoir elements.

Even further, the assembly-connecting means may include a latching member, such as a hook, wherein the corresponding assembly-connecting means may include a recess formed for engaging with said hook. The object is further achieved by a tank assembly adapted to be assembled to a tank, wherein the tank assembly comprises at least two reservoir elements as described above. Further, the tank assembly may include two reservoir elements having a plate element that is sealingly attached to the first or second end of the body element and at least one, preferably at least two further reservoir elements having no such plate element.

The first ends of each reservoir element of the tank assembly may have particularly the same size and shape and the second ends of each reservoir element of the tank assembly may have particularly the same size and shape. It is to be understood, as the body element of the reservoir elements tapers from the first end to the second end, that the size and/or shape of the first and second ends is different.

Particularly, the reservoir elements may have a different height (distance between first and second end). Hence, when combing reservoir elements of different heights, tanks with different volumes can be assembled. Further, different volumes can be achieved by assembling multiple reservoir elements.

As the tank assembly is adapted to be assembled to a tank as described below and includes reservoir elements as described above, all advantages that are described with respect to the tank and the reservoir element can be achieved with the tank assembly, at least when being assembled. Further, the tank assembly allows to transport and store a disassembled tank with little space requirements.

The objects are further achieved with a tank for a bio-pharma process line. The tank comprises a top plate element, optionally at least one sidewall element, and a bottom plate element. The optional at least one sidewall element is a reservoir element having no plate element and the top plate element and the bottom plate element are reservoir elements having a plate element that is sealingly attached to the first or second end of the respective body element.

The top plate element, the optional at least one sidewall element and the bottom plate element are arranged to form a reservoir for receiving at least one biochemical medium. The tank comprises further at least one channel, for guiding the at least one biochemical medium and/or an operating medium, wherein the at least one channel extends within at least one of the top plate element, the at least one sidewall element and/or the bottom plate element. The length of the at least one channel is longer than the thickness of the respective top plate element, sidewall element and/or the bottom plate element.

Generally, all channels and/or reservoirs of the tank may be configured to be self-emptying. I.e. medium that has entered the channel and/or reservoir can flow out of the respective channel and/or reservoir by gravitation.

The assembled tank may comprise one reservoir or multiple (at least two) reservoirs, formed by the top plate element, at least one sidewall element, and a bottom plate element. In case of multiple reservoirs, the reservoirs may be arranged serial or parallel to each other. Medium flowing through serial reservoirs flows through the single reservoirs one after another. Medium flowing through parallel reservoirs flows through the single reservoirs in a parallel fashion. Combinations of serial and parallel arrangement of the reservoirs is also possible.

The tank (and accordingly the reservoir elements) is adapted for being used in a bio-pharma process line, for manufacturing and/or developing bio-pharmaceutical goods and/or for developing and/or testing the commercial manufacturing process of these goods. Further, the tank may be used in other manufacturing lines and may be adapted to be operated automatically and/or manually.

The biochemical medium and/or the operating medium may be any educt or product of the process line, such as sample containing medium, cell containing medium, drug containing medium, buffer medium, acids, bases, and/or the like. The medium may comprise at least one of the following: small molecule API, antibody, drug conjugates, RNA or fragments thereof, rec proteins, viral vaccines, bacterial/microbial processes, virus like particles, viral vectors, ADC, DNA, and/or the like. Further, the operating medium may comprise heating or cooling fluids, pressurized air or gases, and/or the like. For example, in case pressurized air is used as operating medium, the operating medium may serve for driving a biochemical medium through the channel and/or into (or out of) the reservoir of the tank.

The biochemical medium and/or the operating medium may be provided in liquid and/or gaseous form as well as in form of solutions and/or emulsions and/or suspensions.

Particularly, the operating medium, such as pressurized air, may be used to transfer a biochemical medium to and/or out of the tank. Therefore, the tank is operable via the operating medium. For transferring a biochemical medium out of the tank, a positive pressure can be applied to the first tank, by applying operating medium to the tank. Thus, biochemical medium is urged out of the tank, e.g. towards a second tank and/or a filter. For transferring biochemical medium into the tank, a negative pressure can be established, e.g. by removing (operating) medium from the tank. In case pressurized air is used as operating medium, preferably sterile pressurized air is used. Sterile pressurized air can be obtained by guiding pressurized air through a respective sterile filter prior to entering the tank. Operating medium, such as pressurized air can be supplied to or removed from the tank by a respective gas inlet and/or outlet port.

The terms top plate element, side wall element and bottom plate element do not specify the orientation of the tank, when being assembled and in use. Rather, the assembled tank may be used in any orientation. Typically, the bottom plate element serves as a base and can optionally be provided on a stand for adjusting the height of the tank, when being in use/operation. For example, the orientation of the tank can be a standing orientation (i.e. the top plate element is on top), a lying orientation (i.e. a side wall element is on top), or an upside-down orientation (bottom plate element is on top).

Further, the top plate element may serve for supporting the tank and may be adapted to be supported in a support rail, as will be described in greater detail below. In this case, the tank is in a hanging configuration and the bottom plate element may have no contact to the ground. Additionally, support members may be provided that support that tank at the bottom plate element additionally to the hanging support of the top plate element. Said support members may be adapted to be adjustable in height, e.g. by a linear drive, such as a magnetic drive, an electric drive, a pneumatic drive and/or a hydraulic drive. The support members may include a weighing member, allowing to weigh the supported tank. The weight of the respective tank may then be used to control a process line.

Providing reservoir elements, such as a top plate element, optionally at least one side wall element and bottom plate element, that are assembled to a tank and particularly arranged to form a reservoir for receiving at least one biochemical medium facilitates cleaning and maintaining the tank after being used. Thus, the respective reservoir elements or at least some of the reservoir elements can be reused and/or recycled separately. Further, the tank - respectively the elements forming the reservoir - can be easily cleaned and sterilized prior to use.

The at least one channel extends within at least one of the top plate element, the at least one sidewall element and the bottom plate element. Different channels may be provided in different reservoir elements or the same reservoir element, so that each of the reservoir elements (top plate element, the optional at least one sidewall element and/or the bottom plate element) may comprise at least one (or multiple) channels.

Optionally, the medium transfer to and/or from the reservoir of the tank may be achieved exclusively by the at least one channel or multiple channels that extends in the top plate element, the at least one sidewall element and/or the bottom plate element. Thus, the at least one channel and the surface of the reservoir that are in contact with the medium, may be of the same material. Thus, the risk of contamination of the educts and/or products of a bio-pharma process line (i.e. biochemical medium and/or an operating medium) can be reduced and cell growth and/or other bio-chemical process steps are not distorted due to unfavorable combinations of different materials.

Transferring the medium using the channel(s) allows to provide a high lot to lot consistency of the product line. This is, as in conventional fluid connections using e.g. hoses (as e.g. in conventional single use lines) lot to lot variations of the different contact materials occurs. This leads to undesired and unpredicted contamination of the transferred fluid. By transferring the medium using the channel(s) of the tank elements, the number of different contact materials can be reduced to a minimum and lot to lot consistency of the product line can be increased.

The at least one channel may extend in the at least one sidewall element, the top plate element and the bottom plate element. In another example, the at least one channel may extend in the top plate element and in at least one sidewall element. At least one additional channel may extend in the at least one sidewall element, the top plate element and/or the bottom plate element.

Via the at least one channel (or respectively the additional channel), a biochemical medium may be removed from a lower portion of the tank (e.g. via a first end of the channel, being provided in the bottom plate element or in a sidewall element in proximity to the bottom plate element) and guided to an upper portion of the tank (e.g. via a second end of the channel, being provided in the top plate element or in a sidewall element in proximity to the top plate element) e.g. for being transferred into a further tank. Accordingly, a biochemical medium maybe removed from an upper portion of the tank (e.g. via a first end of the channel, being provided in the top plate element or in a sidewall element in proximity to the top plate element) and guided to a lower portion of the tank (e.g. via a second end of the channel, being provided in the bottom plate element or in a sidewall element in proximity to the bottom plate element) e.g. for being transferred into a further tank. Further, an operating medium, such as a heating or cooling medium may be guided within the at least one sidewall element and at least one of the top plate element and the bottom plate element, so as to provide a proper cooling/heating of the assembled tank. The heating or cooling medium may be guided within the top plate element, the at least one sidewall element and/or the bottom plate element without being in contact to the reservoir. Thus, the heating or cooling medium and the bio-chemical medium are separated from each other.

Generally, the tank may serve as reservoir for any educt or product of the process line, such as a biochemical medium and/or an operating medium. Thus, the received medium may be stored and/or transported. Further, the tank may be configured to blend different mediums received in the reservoir of the tank. Further, the tank may serve as bioreactor and may support a biologically active environment. For example, a chemical process which involves organisms or biochemically active substances derived from such organisms can be carried out in said tank. Further, the tank may be designed to grow cells or tissues in the context of a cell culture. The tank may be used as a batch bioreactor, a fed batch bioreactor, a concentrated fed batch bioreactor, or a continuous bioreactor and/or a perfusion bioreactor. Additionally, or optionally, the tank may serve as filtration unit for filtering the medium received in the reservoir of the tank. In this case, a filter may be provided in or on the top plate element, the at least one sidewall element and/or the bottom plate element. Further, the tank may serve for preparing a biochemical medium, such as a buffer medium and/or for carrying out a preparative chromatography. Further, the tank may comprise a cross flow cassette and/or a hollow fibre module, for filtration of virus, cell harvesting and/or ultra or diafiltration.

The reservoir of the tank may have a volume of at least about 10 ml, of at least about 15 ml, of at least about 20 ml, of at least about 50 ml, of at least about 100 ml, of at least about 200 ml, of at least about 250 ml, of at least about 500ml, of at least about 1l, of at least about 2 l, of at least about 5 l, of at least about 10 l, of at least about 20 l, of at least about 50 l, of at least about 100 l, of at least about 200 l, of at least about 500 l, of at least about 1000 l, of at least about 2000 l, of at least about 3000 l, of at least about 4000 l, or of at least about 5000 l. Thus, bio-pharma process lines can be scaled. For example, during development tanks with a small volume are used. Afterwards, during manufacturing, larger tanks are used. Further, tanks of different volumes can be combined in a tank system of a bio-pharma process line.

Tanks with different volumes can be achieved by assembling reservoir elements having a different height (distance between first and second end), and/or by assembling multiple reservoir elements. In case all first ends of each reservoir element of a tank assembly have the same size and shape and the second ends of each reservoir element of the tank assembly have particularly the same size and shape, the reservoir elements can be combined arbitrary to assemble tanks with different volumes. Thus, based on a small number of different reservoir elements, a high number of different tanks can be assembled.

In an embodiment, tanks with different volumes can be provided, using as top plate element and bottom plate element substantially the same reservoir elements and as sidewall elements reservoir elements of different height dimensions or multiple reservoir elements.

For example, the reservoir elements may have a width in the range of 80 mm to 1500 mm, preferably in the range of 200 mm to 1200 mm, even more preferably in the range of 300 mm to 1000 mm and most preferably in the range of 450 mm to 600 mm. Further the reservoir elements may have a height in the range of 150 mm to 2000 mm, preferably in the range of 200 mm to 1300 mm, even more preferably in the range of 500 mm to 1000 mm and most preferably in the range of 450 mm to 650 mm.

The tank may have a height dimension, a depths dimension and a widths dimension, wherein the ratio of hight:depth:width may be of about 2:1:1. This ratio has to be shown to be preferred in case the tank is used as a bioreactor. Other dimensions may be chosen, depending on the desired functionality of the tank.

The tank may comprise a heating or cooling finger that may be inserted into the tank through the top plate element, the at least one sidewall element, and/or the bottom plate element. The heating or cooling finger serves for guiding a tempered heating or cooling medium, for tempering the tank, wherein the heating or cooling medium guided by the heating or cooling finger is separated from the contents of the reservoir. Depending on the degree of heating/cooling, the medium received in the tank may evaporate or condensate.

Further, the tank may comprise at least one port, which can be provided in at least one of the reservoir elements, wherein the at least one port may be associated with a respective channel. The port maybe chosen from a group of port-types, comprising the following port-types: a fluid inlet port, a gas inlet port, a fluid outlet port, a gas outlet port, a cell bleed port, a tank-interconnecting port, an element-interconnecting port, a medium supply port, a medium remove port and/or the like, as described above with respect to the reservoir elements.

The tank may comprise at least one filter, which can be provided in at least one of the reservoir elements, wherein the least one filter may be chosen from a group of filter-types, comprising the following filter-types: a pre-filter, a sterile filter, a bacterial filter, a viral filter, a mycoplasma filter, an ultrafiltration filter, a diafiltration filter, a cell filter, a cell harvest filter, a fluid filter, an air filter, a gas filter, or the like, as described above with respect to the reservoir elements.

The tank may comprise at least one valve, which can be provided in at least one of the reservoir elements. The at least one valve may be associated with the at least one channel. The at least one valve may be a flow control valve, a cutoff valve, a pressure relief valve or a non-return valve, or the like. Further, a tank may comprise multiple valves of the same and/or of different types. Particularly, a valve may be provided at a junction of at least two channel portions. Thus, depending on the valve position, medium can be guided to different channels and/or tanks, as described above with respect to the reservoir elements.

The tank may further comprise an adaptor plate element, wherein the adaptor plate element is mounted on the top plate element. The adaptor plate element may be configured to cover a filter and/or a port of the tank at least partially. In a particular embodiment, a top plate element of a tank and an associated adaptor plate element may form a filter housing. Further, the adaptor plate element may be configured to support an actuating means of a valve.

The adaptor plate element and the top plate element may sandwich a barrier element, wherein the barrier element is part of a clean room. The clean room maybe e.g. a clean room bag or tent. In this case, the adaptor plate element may be arranged outside the clean room and the top plate element, respectively the assembled tank are arranged inside the clean room.

Further, the adaptor plate element may provide access to at least one port of the tank, to at least one filter of the tank and/or to at least one actuating means of a valve of the tank. In case the barrier element is sandwiched between the adaptor plate element and the top plate element, the top plate element as well as the at least one sidewall element and the bottom plate element of the tank can be maintained in the clean room wherein access to the tank from the outside of the clean room is possible via the access provided by the adaptor plate element. Thus, a biochemical medium and/or an operating medium can be supplied to and/or removed from the tank from the outside of the clean room. Further, the at least one valve can be actuated and controlled from the outside of the clean room.

The tank may further comprise at least one connector means for interconnecting the tank (first tank) with a further (second) tank. The connector means may be included in at least one of the reservoir elements, forming the tank. The connector means allows a fast set up of a tank system, comprising several tanks, as the tanks can be interconnected by said connector means. Thus, the assembly of a bio-pharma process line is facilitated and/or speeded up. Additionally, the risk of mal-function and/or leakage due to improper assembly can be reduced. Particularly, the connector means may be adapted for automated interconnection, so that a tank system of a bio-pharma process line can be automated, thereby further reducing the risk of improper assembling.

The connector means may be threaded connector means such as screws and respective nuts and/or belts or straps that interconnect the first and second tank.

The connector means may be a latching connector means, wherein the tank comprises a first latching connector means for directly interconnecting the tank with a further tank, which comprises a corresponding latching connector means. The first latching connector means may be a latching arm including a latching protrusion. The latching protrusion may be adapted to latch with a corresponding latching connector means, which may be formed as latching recess.

The tank, particularly a respective reservoir element of the tank, may alternatively or additionally comprise a second latching connector means, which is configured to latch with an inter-latching connector means, that is adapted to latch with a second latching connector means of a further tank, so that the tank can be directly interconnected with said further tank, via the inter-latching connector means. Said second latching connector means may include a protrusion, such as a nob, that is adapted to latch with an inter-latching connector means, having e.g. a corresponding recess to provide a positive locking. The inter-latching connector means may be a flexible inter-latching connector means, that is tensioned upon latching with the second latching connector means of the tank(s), thereby urging a first tank against a second tank.

The connector means may provide a fluidical connection between the first and second tank. For example, the connector means may be designed and shaped, so that upon latching tank-interconnecting ports of the first and the second tanks engage which each other so that respective channels are aligned and in (sealed) fluid communication. Thus, the assembly of a tank system of a bio-pharma process line can be facilitated and speeded up.

The connector means and in particular the latching connector means may be provided at the top plate element, the at least one side wall element and/or the bottom plate element. Further, the connector means may be provided in a recessed portion of top plate element, the at least one side wall element and/or the bottom plate element so as to prevent the connector means from being damaged during transport and/or storage. Particularly, the connector means may be configured to be operated automatically and/or tool less. The connector means may provide a permanent interconnection of the tanks or a separable interconnection.

At least one connector means of the tank may be configured as a bottom drain. The bottom drain may provide a fluidical connection between the first and second tank and may be provided in the lower half of the first tank, particularly in the bottom plate element. The bottom drain may be configured so that the first tank is self-emptying and/or may emptied by the application of pressure.

Any of the reservoir elements and/or the tank may be sterilizable, by means of autoclaving, ETO gas, x-ray and/or gamma radiation, prior, during or after being assembled. As the tank can be assembled from the reservoir elements (the top plate element, the optional at least one sidewall element and/or the bottom plate element), sterilization is facilitated. In case the tank shall be sterilized after being assembled, the ports can be used for guiding ETO gas or steam into and out of the tank.

Further, the tank may comprise assembly reinforcement means for reinforcing the assembly of the reservoir elements (e.g. the top plate element, at least one of the sidewall elements and/or the bottom plate element) of the tank. The assembly reinforcement means may include a threaded means, such as a threaded rod, a strap and/or a belt. For example, at least two of the reservoir elements may comprise a through opening for receiving a threaded rod. Those reservoir elements can then be assembled by using at least one threaded member, such as a nut, that threadedly engages with the threaded rod. Further, at least one strap or belt may be wrapped around the tank to reinforce the assembly of the reservoir elements. Further, the assembly reinforcement means may improve the tightness of the tank. Thus, leakage can be prevented, and the assembly of the tank can be facilitated.

Further, a tank may be installed in an external frame, that serves as reinforcement means. The external frame may be configured to apply a force on the assembled tank, e.g. by at least one hydraulic plate, thereby reinforcing the assembly of the tank. Further, the external frame may be part of a guide rail system of a handling manipulator, that allows automated control of the tank and/or a tank system.

The tank may comprise multiple side-wall elements, wherein the top plate element, the sidewall elements and the bottom plate element are arranged to form the reservoir. This allows a small packing size of the disassembled tank (i.e. the tank assembly). Generally, the tank may be configured to withstand an inner pressure (without leakage) of at least 2 bars, or of at least 4 bars or of at least 10 bars. A typical operating pressure of a tank/tank system may be in range between 0.1 to 1 bar, preferably in a range between 0.2 to 0.6 bar and even more preferably between 0.25 and 0.5 bars. Particularly, the operating pressure maybe below 0.5 bars, preferably below 0.3 bars.

Depending on the cross section of the reservoir elements the reservoir of the tank may have a substantial rectangular, hexagonal or octagonal cross-section, when seen from the top plate element side. In case of a rectangular cross-section, the tank can be directly interconnected with up to four neighboring tanks. In case of a hexagonal cross-section, the tank can be directly interconnected with up to six neighboring tanks and in case of an octagonal cross-section, the tank can be directly interconnected with up to eight neighboring tanks. In a tank system, tanks of the same and/or of different configurations, such as different cross-sections can be combined. Thus, a process line, such as a bio-pharma process line, can be installed in a very small installation space. Thereby costs, such as clean room costs, can be reduced.

The tank may further comprise at least one pumping means, wherein the pumping means may be separated from the reservoir and/or the at least one channel by a flexible membrane, so as to prevent direct contact between the at least one biochemical medium and the pumping means. The pumping means may be included in a reservoir element.

The tank may further comprise at least one stirring means, wherein the stirring means may be drivable from the outside of the tank. For example, the stirring means may be driven by a magnetic actuator. Using a magnetic actuator allows to drive the stirring means from the outside of the tank and facilitates the sealing, as the magnetic actuator can be separated from the actual stirring means, e.g. by a continuous wall portion. The stirring means may be included in a reservoir element, particularly the bottom plate element. Further, the stirring means may comprise an actuating rod. Said actuating rod may be supported and sealed in a reservoir element (of the tank. The actuating rod can be engageable with a drive mechanism, such as an electric or magnetic drive mechanism, provided on the outside of the tank. Preferably, the actuating rod protrudes from the top plate element. The drive mechanism may be part of a handling manipulator that allows automated control of the tank and/or a tank system.

The stirring means may be supported in the tank by means of the actuating rod. Further, the stirring means may be supported levitatingly in the tank, e.g. by a magnetic bearing. For driving the stirring means, a handling manipulator may couple magnetically to said levitatingly supported stirring means from the outside of the tank and drive the stirring means from the outside of the tank.

Further, the handling manipulator may couple mechanically and/or in any other suitable way, to said stirring means from the outside of the tank and drive the stirring means from the outside of the tank.

Moreover, the at least one handling manipulator may be provided above or underneath the tank(s) in order to drive the stirring means.

The stirring means may comprise at least one stirring member, wherein the stirring member comprises multiple stirring blades. The stirring blades can be provided in different forms, such as in form of a Rushton stirring blade, a pitched blade, a gentle marine blade, or the like. Further, the stirring means may comprise multiple stirring members, being e.g. provided in different levels of the tank. Further, the stirring means may include an integrated sparger.

The stirring means may be operable in a pulsed mode. Thereby waves are created that allow to clean up a filter from a reservoir side. Further, for cleaning up a filter, a further stirring member may be provided that is adapted to rotate in close proximity to the filter. Depending on the type of filter, the reservoir side maybe a permeate side or a retentate side of the filter.

The tank may further comprise at least one blending means, such as a fluid deflection plate, which may be integrally formed with either one of the reservoir elements. Further, the blending means may be a separate means that is provided in the reservoir of the tank. The at least one blending means may be associated with an inlet port of the tank/the respective reservoir element.

The tank may further comprise at least one means to avoid foaming of the at least one biochemical medium and/or operating medium. The at least one means to avoid foaming may reduce the velocity, the fall height and/or the flow properties of the at least one biochemical medium and/or operating medium. The at least one means to avoid foaming may be a fluid deflection plate and/or a channel (e.g. a rigid tube) as described above. In case of being a channel, the channel opening maybe directed towards one of the sidewall elements, the top plate element and/or the bottom plate element so that the biochemical medium and/or operating medium is deflected when entering the reservoir through the channel. The at least one means to avoid foaming may be integrally formed with one of the reservoir elements. Further, the at least one means to avoid foaming may be a separate means that is provided in the reservoir of the tank. The at least one means to avoid foaming may be associated with an inlet port of the tank.

The tank may further comprise at least one a cell-harvest-means. The cell-harvest means may include a filter and/or a filter cartridge that can be coupled or integrated in a reservoir element, particularly the bottom plate element of the tank. The cell-harvest-means may comprise medium remove port (also referenced as cell bleed port), for removing the medium or parts of the medium (such as cells) contained in the reservoir of the tank. The medium remove port may also be provided in the bottom plate element and/or the at least one side wall element. In particular, the medium remove port can be provided anywhere on a reservoir-side of a filter of the cell-harvest-means for removing retentate, such as cells, and/or on the opposite side, for removing filtrate (permeate). Further, the cell-harvest-means may include an (operating) medium supply port, that allows to rinse a filter of the cell-harvest-means.

Further, the tank may be associated with at least one magnet, wherein the at least one magnet may be used for cell selection, cell activation, transduction and/or expansion. The at least one magnet may be arranged directly on the top plate element, the at least one sidewall element and/or the bottom plate element. The at least one manipulator may be adapted to arrange and/or remove the at least one magnet.

An operating medium supply port may be provided on the permeate side of the filter, or a filtrate side, respectively. Thus, when positive pressure (gaseous or fluidic) is applied on the permeate/filtrate side of the filter, e.g. via the (operating) medium supply port, the retained portion of the feed or retentate can be transferred back into the tank, out of the medium remove port and/or out of a waste port. Further, for transferring retentate back into the tank and/or out of the medium remove port a negative pressure can be applied on the retentate side of the filter.

Applying positive pressure on the permeate/filtrate side of the filter (preferably by providing a pressurized operating medium) and/or negative pressure on a retentate/upstream side of the filter, allows to flush the filter. Thus, a blocked filter, e.g. a cell filter, can be blown out.

Additionally, applying positive pressure (gaseous or fluidic) on the permeate side of the filter or a filtrate side, respectively, e.g. via the (operating) medium supply port, permeate/filtrate can be urged into a permeate channel or a respective filtrate channel. Thus, permeate/filtrate can be guided back into the reservoir and/or to a further tank. Preferably, positive pressure is applied by providing sterile pressurized air on the permeate side or the filtrate side, respectively.

The tank may further comprise at least one cartridge for chromatography, so as to allow carrying out a preparative chromatography. Further, the tank may comprise at least one resin means, a membrane absorber, and/or the like. The tank may further comprise at least one cross-flow cassette, so as to allow carrying out crossflow filtration or tangential flow filtration within the tank.

The tank may further comprise at least one hollow-fibre means for cell harvesting, diafiltration, micro-filtration, ultra-filtration, and/or the like. The hollow-fibre means may be provided in a cartridge that can be integrated in the reservoir of the tank or that can be arranged outside of the reservoir of the tank. For example, the hollow-fibre means may be coupled to at least one of the reservoir elements of the tanks, using respective ports. Further, the tank may comprise multiple hollow-fibre means. Particularly, a tank may be used for increasing the cell concentration of a cell containing solution, e.g. by filtering the solvent. This allows to harvest cultivated cells.

A reservoir element, such as the at least one sidewall element or the bottom plate element may be integrally formed with either one of the above-mentioned filter, cell-harvest-means, cartridge for chromatography, cross-flow-cassette, resin means, hollow-fibre means, and/or any other fluid storing or guiding component.

Particularly, any of one of the above-mentioned filter, cell-harvest-means, cartridge for chromatography, cross-flow-cassette, resin means, hollow-fibre means, and or any other fluid storing or guiding component may include a capsule, wherein the capsule houses the respective filter, means, cassette, and/or component. Further, the capsule and at least one of the sidewall elements and/or the bottom plate element may be integrally formed, wherein the at least one channel of the tank may extend within the capsule (i.e. at least one of the sidewall elements and/or the bottom plate element). Alternatively, the capsule may be connected to at least one of the reservoir elements. This connection may be permanent or reversible.

The tank may further comprise at least one a rupture disc, which may be provided in a reservoir element. The rupture disk may be formed of different materials, such as stainless steel, graphite, silicon, plastics, and/or the like. The rupture disc maybe arranged to block e.g. an outlet port, or a medium remove port of the tank. In case the pressure inside the reservoir exceeds a predefined threshold (e.g. due to a blocked filter), the rupture disc ruptures and medium can be guided out of the reservoir via the respective port, without damaging the tank. Additionally, or alternatively a pressure relief valve may be provided that allows for releasing excess pressure from the reservoir of the tank.

The tank may further comprise at least one bag, wherein the bag may line the inner wall of the reservoir formed by the reservoir elements. Thus, the reservoir of the tank can easily be prepared for receiving further fluids, e.g. by exchanging the bag.

The tank may be connectable to at least one sensor or a sensor module, comprising multiple sensors, wherein the at least one sensor and the sensors of the sensor module are chosen from the group of a pH sensor, a temperature sensor, a dissolved oxygen sensor, a biomass sensor, a foam sensor, a pressure sensor, a flow sensor, an O₂ sensor, an N₂ sensor, a CO₂ sensor, or the like. Further, the at least one sensor may be a spectroscopy means, such as RAMAN, NIR and/or UV spectroscopy means. Further, the sensor or sensor module may be connected to the tank and in particular to a reservoir element of the tank. Further, the sensor or sensor module may be a single use sensor/sensor module. Moreover, the flow sensor may be an ultrasonic sensor, which is optionally arranged at the at least one channel of the tank. Further, the sensor or sensor module may be cleanable and optionally sterilizable.

For measuring pressure within a tank and/or a channel thereof, a channel may include a flexible membrane. This membrane can be associated with a pressure sensor that is adapted for the pressure inside the tank and/or the channel. Further, the pressure within the tank and/or the channel may be measured, using a hydrophobic filter, such as a vent filter, that is installed in the top plate element. This filter can be separated from the reservoir of the tank by means of a valve. For measuring the pressure, the valve can be opened.

Being able to measure the pressure in a tank and/or a channel thereof, allows for leakage testing of the tank and/or for non-destructive integrity testing of filter(s) provided in the tank. Prior to testing, the tank is pressurized, e.g. by applying pressurized air. Subsequently pressure drop can be measured and compared to respective predefined pressure values. Thus, leakage can be detected. In case the measured pressure drop exceeds the predefined pressure values, the test is not passed.

For testing integrity of a filter, all input- and output channels of the tank should be closed, e.g. by using respective valves. Closing the valves is preferably carried out by a handling manipulator, i.e. automatically. Further, the filter should be wetted prior to testing. Wetting the filter can be achieved by opening a wetting channel that is associated with the filter to be tested. The wetting channel may guide a buffer solution to the filter, that wets the filter. Subsequently, the tank, particularly a permeate side (filtrate side) and/or a retentate side (upstream side) of the filter, may be pressurized and pressure drop over time can be measured. For pressurizing positive and/or negative pressure may be applied so that there is a differential pressure applied on the filter. The measured pressure drop can be compared to a predefined threshold to decide, whether the integrity test is passed or not. This integrity test can be performed for every filter of the tank. Integrity can then be tested e.g. using a pressure hold test, a pressure drop test and/or a forward flow test.

For testing integrity, the following steps can be carried out:
a) The filter(s) that shall be tested is wetted with a wetting fluid, e.g. by applying a buffer solution. The buffer solution may be applied through a buffer containing cartridge or tank, preferably by using the handling manipulator, i.e. automatically. For wetting the filter(s) a channel that fluidically connects the buffer containing cartridge or tank with the tank that contains the filter to be tested is opened, e.g. by opening a respective valve and/or by connecting the buffer containing cartridge or tank to the tank that contains the filter to be tested. Operating the valve(s) and connecting the buffer containing cartridge may be carried out by the handling manipulator.

Further it may be monitored, whether wetting is completed. Completion of the wetting step can be detected e.g. by detecting permeate and/or filtrate that has travelled through the filter to be tested. This can be done by weighing, by providing a fluid sensor, such as a capacitive sensor, and/or the like. Additionally, or alternatively, the wetting fluid and respectively pressure, maybe provided for a predetermined period of time that guarantees a proper wetting of the filter(s). Excess wetting fluid can be removed via a waste channel and e.g. guided to waste tank. Further, if needed back pressure can be applied via the valves for improving the wetting of the filter.
b) After wetting is completed, e.g. the permeate side (filtrate side) and/or a retentate side (upstream side) of the filter is pressurized, preferably by applying sterile pressurized air. Prior to pressurizing, all valves that would allow pressurized air to leave the side of the filter that is pressurized should be closed, preferably by a respective handling manipulator.

For testing membrane filters, typically the upstream side of the filter is pressurized. On the filtrate side of the filter a channel may be opened, e.g. a waste channel, preferably by actuating a respective valve. This allows pressurized air that has travelled though the filter to be removed from the filtrate side.

For testing a cross flow cassette and/or a hollow fibre module pressure may be applied on the retentate side, preferably via a retentate outlet channel of the tank The retentate outlet channel maybe openable/closable by an associated valve. The feed channel(s) of the cross flow cassette and/or a hollow fibre module may be closed for integrity testing and the permeate channel(s) of the cross flow cassette and/or a hollow fibre module may be opened for integrity testing. On the permeate side of the filter a channel may be opened, e.g. one of the permeate channels, and connected to a waste channel, preferably by actuating a respective valve. This allows pressurized air that has travelled though the filter to be removed from the permeate side.
c) After or during pressurizing, the pressure drop over time can be measured and compared to a predetermined threshold. Preferably, measuring is started after a predefined stabilization time. In case the pressure drop exceeds the threshold, the test is not passed.

After testing, the pressurized air may be removed from the tank via an outlet port.

Integrity testing is extremely difficult in conventional bag-based process lines, as the bags and/or hose connections expand due to the applied pressure. And therefore, the test result is distorted. Thus, in conventional bag-based process lines expensive helium leakage tests are required. Further, helium leakage testing is complex, and typically cannot be carried for interconnected bags of a conventional bag-based process lines, or even an entire bio-pharma process line. Particularly, interconnections of bags cannot be tested. Thus, being able to use integrity testing in a readily assembled process line and/or a readily assembled tank significantly reduces the risk of leakage. Further, costs for operating a process line.

At least one reservoir element of the tank (top plate element, optional at least one sidewall element and/or bottom plate element) may comprise a sensor module connection portion that allows to connect the sensor or a sensor module, comprising multiple sensors to the tank. The sensor and/or sensor module may comprise a wired or wireless data transfer unit for transferring achieved sensor data to a control unit. Further, the sensor and/or sensor module may comprise a rechargeable battery and/or a power interface for powering the sensor and/or the sensor module. The power interface may be wired or wireless, such as an inductive or capacitive power interface. Further, the sensor and/or sensor module may be configured for multi-use. Particularly, the sensor and/or sensor module may be sterilizable.

The sensor and/or sensor module may be connected with a respective handling manipulator. The connection may be wired and/or wireless. Thereby data may be transferred between the sensor and/or sensor module and the handling manipulator. Further, with said connection, the sensor may be powered by means of the handling manipulator.

The reservoir elements and the tank may be free of electric or electronic parts but can be connected to the same (e.g. a sensor or a sensor module). Thus, the reservoir elements and the tank can be easily recycled.

Further, the object is achieved by a tank system, comprising multiple tanks as described above. A first tank of the tank system is interconnectable with a second tank by the at least one connector means, when the second tank is arranged adjacent to the first tank. Further, at least one of the one or more channels of the first tank are fluidically connected to respective channels of the second tank, when the first tank is interconnected with the second tank. Thus, a process line, such as a bio-pharma process line can be set up easily and automated. Particularly, the number of hose- or pipe based fluidic connections can be significantly reduced, compared to conventional process lines.

Particularly, a first of the tank system may be directly interconnectable with a second tank by the at least one connector means, when the second tank is arranged directly adjacent to the first tank. Alternatively, or additionally hose-based fluid connections can be used.

To facilitate the connection, the top plate elements, particularly the plate elements, of the first and second tank may protrude laterally over the sidewall elements. Hence, connecting the tanks is facilitated, as the sidewall elements do not contravene the connection. Further, the bottom plate elements may protrude laterally over the sidewall elements. Thus, the tanks may be additionally connected via the bottom plate elements.

Further, a height dimension of the first tank may be smaller than a height dimension of the second tank. In order to compensate for the height difference, the tank system may comprise a support rail, wherein the support rail being adapted to support at least two tanks. The support rail may engage with the top plate elements of the respective tanks (here e.g. first and second tank), so that top plate elements of the tanks with different height dimensions are arranged on the same level, when the tanks are supported by the support rail. Additionally, or alternatively, the support rail may engage with sidewall elements of the respective tanks, so that an (additional) support of the tanks is provided. The support rail may include at least one weighing member, allowing to weigh the supported tanks individually. The weight of the respective tanks may then be used to control a process line formed by the tanks.

It is to be understood, that the support rail can be utilized for tanks of the same height dimensions as well. Particularly, the support rail may at least partially be included in a clean room bag.

Thus, all top plate elements of the tank system can be arranged at substantially the same height. This allows to mount respective adaptor plate element on top of the plate elements, wherein the adaptor plate elements are also arranged at substantially the same height. Thus, ports of the tanks, filters of the tanks and/or actuating means of valves of the tanks can be accessed and operated at substantially the same height. Thereby automated actuation and operation of the tank system is facilitated. Further, in case the adaptor plate element and the top plate element sandwich a barrier element that is part of a clean room, the clean room can have a substantially flat upper surface and there is no need for a complex geometry.

Still further, at least one adaptor plate may be configured to be mounted on multiple top elements of different tanks, thereby strengthening the interconnection of said tanks.

Further, a volume of the first tank may be smaller than a volume of the second tank and the first tank may be adapted to be installed on top of the second tank. The first and second tanks may be fluidically connected, wherein the fluid connection (channel) may be controlled by a valve. The first tank may serve to provide an operating medium and/or a biochemical medium to the second tank. As the first tank is installed on top of the second tank, it is possible to transfer fluid from the first tank to the second tank by using gravitation. Thus, the use of a pump can be avoided.

Further, medium can be transferred from a first tank to a second tank by pressurizing at least one of the tanks. In case medium shall be transferred from the first to the second tank, a positive pressure can be applied to the first tank and/or a negative pressure can be applied to the second tank. Thus, medium is urged from the first tank towards a second tank. In case a filter is provided between the first tank and the second tank (e.g. in an interconnection channel, and/or in a top plate element of the second tank, in case the first tank is installed on top of the second tank), filtration can be controlled by controlling the pressure level in the first and/or second tank. A positive pressure can be established by providing (operating) medium, such as pressurized air, to the tank. A negative pressure can be established by removing (operating) medium from the respective tank. In case pressurized air is used as operating medium, preferably sterile pressurized air is used. Sterile pressurized air can be obtained by guiding pressurized air through a respective sterile filter prior to entering the tank.

Further, the tank system may comprise a connection plate element. Said connection plate element may include at least one channel and may be adapted to interconnect tanks that are not provided directly adjacent to each other. Using a connection plate element facilitates interconnecting spaced apart tanks. Those spaced apart tanks can e.g. sandwich one or more further tanks. Further, a connection plate element may be configured to multiplex or demultiplex a medium flow. For example, the connection plate element may contain a channel junction or a channel manifold. Thus, a medium received from a first tank can be supplied to multiple other tanks (multiplexing). Further, different mediums can be mixed within the connection plate element and can then be jointly supplied to a single tank (demultiplexing). Further, the connection plate element may be integrally formed with any one of the top-plate element, the at least one side wall element and/or the bottom plate element.

Moreover, the tank system may comprise an adapter. Said adapter may be adapted to interconnect the first tank and the second tank. Optionally the adapter is adapted to interconnect the first tank and the second tank by means of the at least one connector means.

The adapter may include at least one channel and at least one fluidic module. The at least one channel of the adapter may be fluidically connected to a respective channel of the first tank and a respective channel of the second tank, when the adapter interconnects the first tank and the second tank.

The at least one fluidic module may serve to filter, mix, separate and/or activate a medium flow (biochemical medium and/or operating medium). The at least one fluidic module may be at least one of the following: a crossflow cassette, a crossflow hollow fiber module, a hollow fiber filter, a resin capsule, a filter capsule, and/or a magnetic tube. It is to be understood, that the adapter may include multiple fluidic modules of the same type and/or of different types.

Further, the at least one fluidic module may be replaceable. Thus, maintenance may be facilitated. Particularly, the at least one fluidic module may be replaceable by another type of fluidic module. Thus, the functionality of the adapter may be adapted with reduced effort. Further, the at least one fluidic module may be replaceable by the same type of fluidic module. Thus, e.g. a consumed filter can be easily replaced.

Further, the adapter may be adapted to interconnect tanks that are not provided directly adjacent to each other. Using the adapter facilitates interconnecting spaced apart tanks. Those spaced apart tanks can e.g. sandwich one or more further tanks. Further, the adapter may be configured to multiplex or demultiplex a medium flow, so as the connection plate element.

The system may further comprise at least one clean room bag. Further, some objects are achieved by said clean room bag. The clean room bag is configured to receive at least one tank and to provide a clean room environment for the at least one tank when the at least one tank is received in the clean room bag. The clean room bag comprises at least one foil portion, that is adapted to be sandwiched between an access plate element of the tank, e.g. a top plate element of the tank, and an adaptor plate element, so as to define at least one access point to provide access to the tank from the outside of the clean room bag, via the access plate element. Further, the at least one tank may be accessible with a handling manipulator via the access plate element, from the outside of the clean room bag.

The clean room bag can be installed in almost every environment, e.g. in a conventional factory hall. Thus, expensive and time-consuming planning and constructing of pharma-process line plants can be avoided. Thus, the bio-pharma process line can be set up very quickly. Further, as the clean room bag as such provides at least clean room conditions, no expensive conventional clean rooms have to be installed. Further, in case of a single-use clean room bag, expensive and time-consuming cleaning and testing can be avoided.

Preferably, the clean room bag is a flexible bag that requires little storing space, when not in use. The clean room bag can be pre-sterilized and inflated/unfolded when in use. Thus, a sterile or at least clean room environment can be provided.

The clean room bag provides at least clean room conditions. The clean room bag may provide at least ISO 8 (ISO 14644-1 and ISO 14698) conditions. Depending on the required environment, clean room conditions up to ISO 1 are also possible. More preferably, the clean room bag provides a sterile environment for operating the tank(s). The clean room bag may be sterilizable, e.g. by ETO, gamma sterilization, x-ray sterilization or autoclaving.

The system may further comprise at least one adaptor plate element, wherein the adaptor plate element is associated with at least one tank. Particularly the adaptor plate element may be associated with a single tank or with multiple tanks. The adaptor plate element can be installed on the at least one tank, so that one of the at least one foil portions of the clean room bag is sandwiched between an access plate element of the tank and the adaptor plate element. The access plate element may be the top plate element, the at least one sidewall element or the bottom plate element. Particularly preferred, the top plate element is the access plate element.

Further, the adaptor plate element is configured to define at least one access point to provide access to the tank from the outside of the clean room bag, via the access plate element.

Accordingly, the tank can be provided in a clean room or sterile environment and at the same time can be accessed from the outside of the clean room bag. Thus, the handling manipulator and other control or operating devices can be provided outside of the clean room bag. Thus, the size of the clean room bag can be minimized.

The adaptor plate element may be configured to cover a filter and/or a port of the tank at least partially. Thus, medium (bio-chemical and/or operating medium) can be transferred to and/or removed from the tank from the outside of the clean room bag. A foil portion of the clean room bag that is sandwiched between the access plate element of the tank and the adaptor plate element may be provided removably and/or pierceable in an area of the filter and/or port of the tank. Thus, access to the tank can be provided from the outside of the clean room bag.

For accessing a tank from the outside of a clean room bag, a handling manipulator may carry out the following steps:
Step 1: Install an adaptor plate element on the at least one tank, so that one of the at least one foil portions of the clean room bag is sandwiched between the access plate element of the tank and the adaptor plate element. Optionally, remove a cover element, such as a cap, from a through hole (access point) of the adaptor plate element.
Step 2: Perforate the at least one foil portion of the clean room bag with a perforating means, such as a knife, needle, laser and/or the like. The perforation may be carried out in a way, that a foil portion is separated from the clean room bag.
Step 3: Optionally, gripping the separated foil portion using a gripping means, such as a vacuum gripper of the handling manipulator.
Step 4: Optionally, welding the remaining foil portion with the adaptor plate element and/or the top plate element, preferably by using the handling manipulator.
Step 5: Optionally, placing a connector means, such as a quick connector means on the adaptor plate in an area of the removed/perforated foil portion. The adaptor plate element may be configured to support an actuating means of a valve and/or an operating means (such as a stirring means) of the tank, and wherein the foil portion may be provided removably and/or pierceable in an area of the actuating means of a valve and/or an operating means of the tank to provide access to the tank. Access may be provided using the handling manipulator, as described above. Thus, a valve or an operating means, such as a stirring means, can be operated from the outside of the clean room bag.

Further, the clean room bag may be adapted to sealingly couple to an assembly room that servers for assembling at least one tank, wherein the clean room bag may be further configured to receive the at least one assembled tank from the assembly room. The assembly room may also provide a clean room and/or sterile environment for assembling the tank(s). Thus, the risk for contamination of the tank(s) during assembly is reduced.

The clean room bag may be a sterile bag, that is optionally a single-use bag. The clean room bag may be initially sterilized or maybe sterilizable at the site of the bio-pharmaceutical goods manufacturer. By providing a single-use bag, the risk for contamination of the tank(s) is further reduced. Still further, cleaning the clean room bag is not required after use.

The clean room bag may be held by a support structure. The support structure may be a rigid support structure, such as a frame. Alternatively, the support structure may be inflatable. It is also possible, that the clean room bag as such can be inflated. Thus, a mobile and self-standing clean room can be provided.

The clean room bag may comprise a multi-ply outer shell, wherein a first ply of the shell is the outermost ply, and wherein a second ply of the shell is the innermost ply. The space surrounded by the second ply of the shell may be pressurized with a pressure being lower than a pressure provided between the first and the second ply of the shell. A colored gas may be provided between the first and the second ply of the shell. Thus, when the second ply of the shell would leak, colored gas would enter the space surrounded by the second ply. This can be detected and hints at entry of contaminants in the sterile and/or clean room environment.

The clean room bag may include a sealable opening that is configured for receiving assembled tanks, wherein the sealable opening of the clean room bag may be configured to open jointly with a corresponding sealable opening of an assembly room, when the clean room bag is sealingly coupled to the assembly room. Further, a sealable opening may be used to remove tanks from the clean room bag. The clean room bag may include multiple sealable openings. Thus, transferring assembled tanks from the assembly room to the clean room bag or out of the clean room bag is facilitated.

The sealable opening of the clean room bag may be covered by at least one cover element. The corresponding sealable opening of the assembly room may be covered by at least one corresponding cover element. The at least one cover element and the at least one corresponding cover element are configured to couple with each other to open jointly.

For example, the sealable opening and the corresponding sealable opening may be provided as sliding doors. The at least one cover element/corresponding cover element maybe a door leaf of the respective sliding door. Coupling the cover element(s) with the corresponding cover element(s) can be achieved e.g. by magnetic means, positive locking means, or any other coupling or a locking means. Preferably, magnets (permanent of electric) are distributed at least on an edge portion of the cover element(s)/corresponding cover element(s). This allows coupling the cover element(s) with corresponding cover element(s) in a defined manner. Thus, the non-sterile outer faces of the cover element(s)/corresponding cover element(s) cover each other after coupling. Thus, the risk for contaminating the assembled tank(s) when being transferred through the sealable openings from the assembly room to the clean room bag can be further reduced.

The cover element(s)/corresponding cover element(s) are movable with respect to the clean room bag and/or the assembly room. The cover element(s)/corresponding cover element(s) and the clean room bag /assembly room maybe sealed, e.g. by at least one brush seal, a lip seal and/or the like. Even more preferably, the cover element(s)/corresponding cover element(s) may be connected with the clean room bag, or the assembly room via a flexibel portion, such as a sealing foil portion. The flexibel portion may be connected to the cover element on a first side and to the clean room bag on a second side. Likewise, the flexibel portion maybe connected to the corresponding cover element on a first side and to the assembly room on a second side. Thus, the sealable opening can be opened/closed without providing a gap between the cover element and the clean room bag and/or the corresponding cover element and the assembly room, respectively. Thus, the contamination risk can be further reduced.

The clean room bag may further comprise a sealing frame or may be associated with a sealing frame. The sealing frame may be configured to frame the sealable opening of an assembly room. Thus, an additional seal is provided that seals the opening from the environment, when being opened. Thus, the contamination risk can be further reduced. The sealing frame may be a separate frame, may be provided on the assembly room.

The system may further comprise a UV-light source. The UV-light source may be associated with the sealable opening the assembly room. Thus, possible contaminants on the outer surface of the sealable opening can be removed/destroyed prior to and/or during opening the sealable openings. The at least one UV-light source may be provided within the sealing frame. Preferably, the UV-light source is configured to illuminate the sealable opening(s) from different orientations. Thus, contaminants can be removed/destroyed effectively.

The sealing frame may further comprise a port that allows evacuating a space between the sealing frame and the closed sealable openings the assembly room prior to opening the sealable openings. Thus, particles and/or contaminants can be effectively removed, and the risk of contamination can be further reduced.

The clean room bag may comprise at least one rail for guiding at least one received assembled tank within the clean room bag. Thus, positioning the assembled tank(s) within the clean room bag is facilitated. For example, a first tank may be set on the rail. A second tank can be interconnected with the first tank and also set on the rail. Thereby, the first tank can be guided further into the clean room bag. Repeating this, an entire process line can be assembled and guided into the clean room bag.

The rail may be a support rail for guiding at least one received assembled tank within the clean room bag. The support rail is adapted to support at least two tanks, wherein the support rail may engage with the top plate elements and/or sidewall elements of the respective tanks, so that the top plate elements of tanks with different height dimensions are arranged on the same level, when the tanks are supported by the support rail. In this case, the tanks are in a hanging configuration and the bottom plate elements may have no contact to the ground. Additionally, support members may be provided that support that tank(s) at the bottom plate element additionally to the hanging support of the top plate element.

The support rail may be formed from multiple support rail sections, so that the clean room bag is foldable. Particularly, the support rail sections may be made from a plastic material and may be injection moulded. In a particular embodiment, the foil portion and the support rail sections are fixedly adhered to each other (e.g. welded or glued) or even integrally formed. To enhance the load capacity of the support rail sections, those sections may include a metallic insert.

Further, the support rail sections can be configured to receive a corresponding support rail of a frame, which may be part of a handling manipulator. The frame may include a rigid corresponding support rail, such as a metallic support rail. The support rail sections can be formed so as to fit the corresponding support rail of the frame. Further, the support rail sections may include a latching means, that allow to engage the support rail sections with the corresponding support rail of the frame, preferably by snap fit.

Further, improving the guiding of the tanks in the support rail, the top plate elements, the sidewall elements and/or the support rail, respectively the support rail sections may include support wheels or support rollers. The support rollers/wheels may be included in a roller member, that is connectable with a respective top plate element and/or side wall element of the tank. The support rollers/wheels are then guided in the support rail for supporting and guiding the tank in a hanging configuration.

The support rail / support rail sections may include at least one weighing member, allowing to weigh the supported tanks individually. The weight of the respective tanks may then be used to control a process line formed by the tanks.

The object is also achieved by a method for assembling a tank, wherein the method comprises the following steps: providing a top plate element, optionally providing at least one sidewall element, providing a bottom plate element and assembling the top plate element and the bottom plate element and optionally the at least one sidewall element to form a reservoir for receiving at least one biochemical medium.

The assembling may be fully automated. Further, the assembling is preferably carried out in a clean room or even a sterile environment. Further parts of the tank (as described) above may also be assembled.

### Brief Description of the Figures

In the following, the accompanying figures, that schematically show embodiments of the invention are described. Here,
- Fig. 1A: schematically shows an exploded view of a reservoir element;
- Fig. 1B: schematically shows an exploded view of a further reservoir element;
- Fig. 2: schematically shows a reservoir element;
- Fig. 3: schematically shows two reservoir elements, being stacked within each other;
- Fig. 4: schematically shows a reservoir element, which can be used as a bottom plate element of a tank;
- Fig. 5: schematically shows a reservoir element, which can be used as a top plate element of a tank;
- Fig. 6: schematically shows an assembled tank;
- Fig. 7A to C: give an overview of assembled tanks, having different volumes;
- Fig. 8: schematically shows a tank system;
- Fig. 9: schematically shows an enlarged view of a reservoir element;
- Fig. 10: schematically shows an enlarged view of a further reservoir element;
- Fig. 11: schematically shows further aspects of a tank system;
- Fig. 12: schematically shows a clean room bag;
- Fig. 13: schematically shows a clean room bag, and
- Fig. 14: schematically shows a flow diagram of a method for assembling a tank.

### Detailed description of the Figures

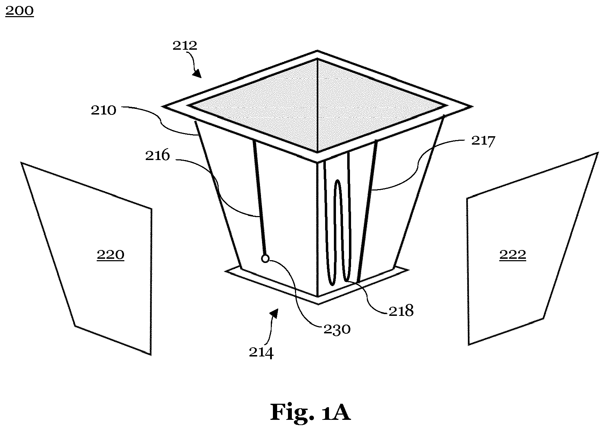

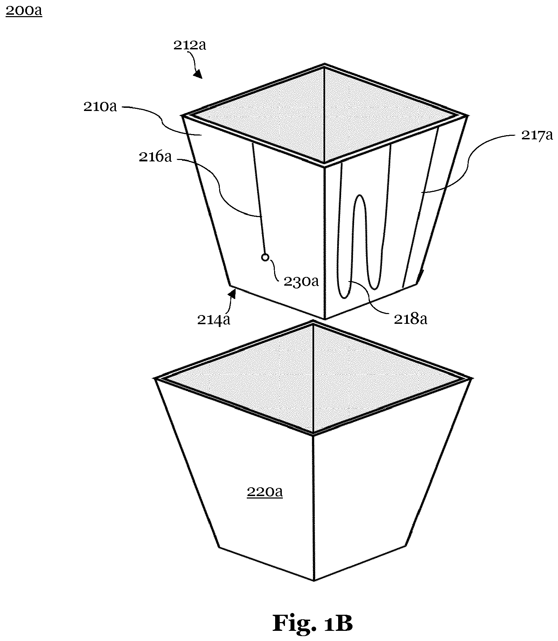

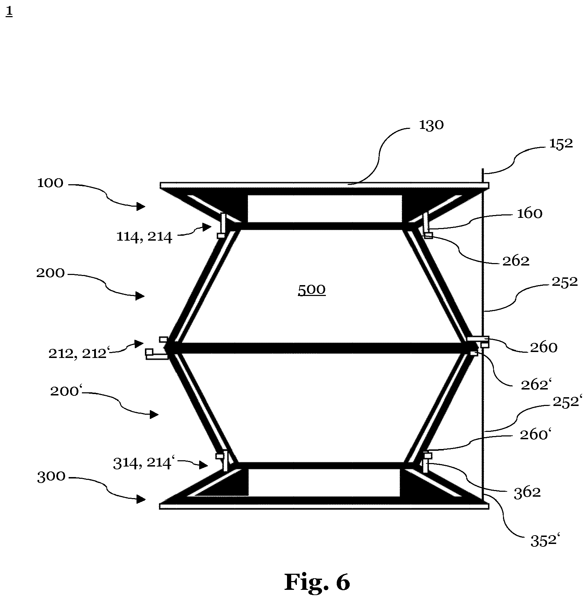

Figs. 1A and 1B schematically show exploded views of different reservoir elements 200, 200a for a tank 1 (cf. Fig. 6) of a bio-pharma process line. The reservoir element 200 includes a body element 210, and multiple shell elements 220, 222 (cf. Fig. 1A). The reservoir element 200a includes a body element 210a, and a single shell elements 220a (cf. Fig. 1B). The body elements have a first end 212, 212a and a second end 214, 214a being opposite the first end, each of the first and second ends including an orifice, and wherein the body elements 210, 210a taper from the first end 212, 212a to the second end 214, 214a, wherein an inner volume is defined between the first and second ends.

The body elements 210, 210a shown in Figs. 1A and 1B have a polygonal cross-section (quadrangular) in a plane parallel to the first end 212, 212a. Hence, the body element forms four substantially flat lateral wall portions. In Fig. 1A, the respective shell elements 220, 222 are likewise plate shaped. Alternatively, the shell element 220b may also have a polygonal cross-section, as shown in Fig. 1B. In a further alternative, the shell element may be formed to cover only some, for example at least two, lateral wall elements of the body element 210, 210a, when being attached.

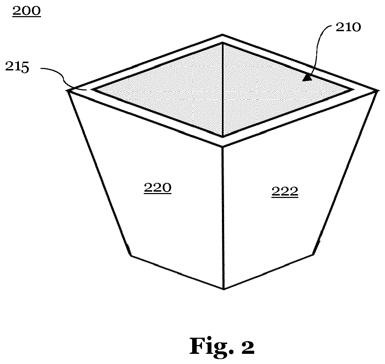

The body element and/or the at least one shell element include at least one groove 216, 217, 218. Here, the grooves 216, 217, 218 are included in the body element. Groove 217 extends from the first end to the second end, wherein groove 216 extends from the first end 212 to a port 230 of the reservoir element. Port 230 may be a fluid inlet port. Groove 218 begins and ends at the first end 212, while including several windings. In Fig. 2, the shell elements 220, 222 are fixedly attached (e.g. welded or glued) to the body element 210 so that the body element 210 and the shell elements 220, 222 sandwich the respective grooves 216, 217 in between forming channels. These channels are adapted for guiding at least one biochemical medium and/or an operating medium. For example, the channel formed by groove 216 may be used to guide a biochemical fluid into the inner volume of the reservoir element (via port 230). The channel formed by groove 217 is adapted to transfer a fluid from the first end to the second end and the channel formed by winding groove 218 may be used as a heating/cooling channel.

Further, the first end 212 of the reservoir element 200 includes a sealing face 213, for sealingly connect the reservoir element 200 to a further reservoir element, as exemplarily depicted in Fig. 6.

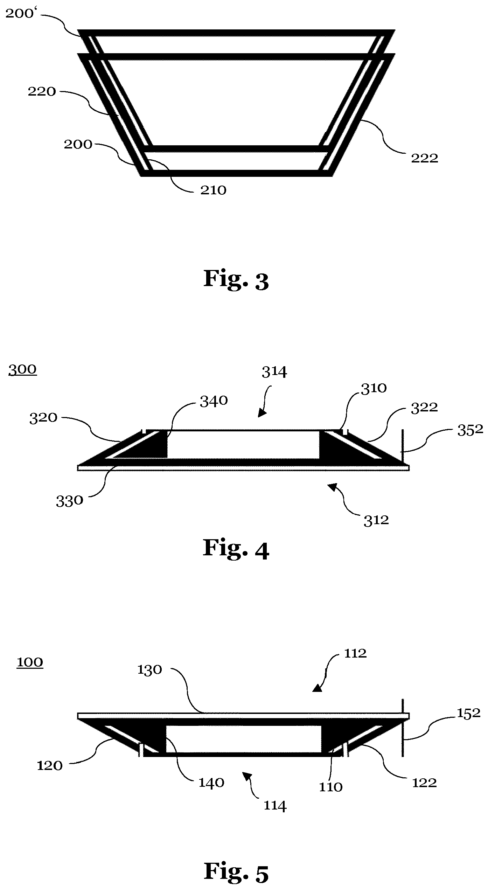

Fig. 3 schematically shows two reservoir elements 200, 200', being stacked within each other. Particularly, the inner volume and the first orifice of said reservoir elements 200, 200' are configured so that the reservoir element 200 is adapted to receive a further reservoir element 200' at least partially, so that multiple reservoir elements are stackable within each other. Thus, storage space can be minimized.

Fig. 4 schematically shows a reservoir element 300, which can be used as a bottom plate element of a tank. This reservoir element 300 includes a tapered body element 310 and multiple shell elements 320, 322 that are fixedly attached to the body element.

Further, the reservoir element 300 includes a plate element 330 being sealingly attached to a first end 312 of the body element 320, so as to cover the respective orifice. Said plate element 330 may include multiple layers and may particularly include at least one channel (not shown), wherein the at least one channel may be in communication with the channel formed by the body element 310 and the at least one shell element 320, 322. The reservoir element 300 further comprises an insert 340 that covers an undercut of the inner volume at least partially. Hence formation of a dead volume is prevented. Still further, the reservoir element includes an actuating means 352, which may be used of opening/closing a valve (not shown). Said valve may be a magnetic valve.

Fig. 5 schematically shows a reservoir element 100, which can be used as a top plate element of a tank. The reservoir element 100 includes a plate element 130 being sealingly attached to a first end 112 of the body element 120, so as to cover the respective orifice. Said plate element 130 may include multiple layers and may particularly include at least one channel (not shown), wherein the at least one channel may be in communication with the channel formed by the body element 110 and the at least one shell element 120, 122. The reservoir element 100 further comprises an insert 140 that covers an undercut of the inner volume at least partially. Hence formation of a dead volume is prevented. Still further, the reservoir element 100 includes an actuating means 152, which may be used of opening/closing a valve (not shown). Said valve may be a magnetic valve.

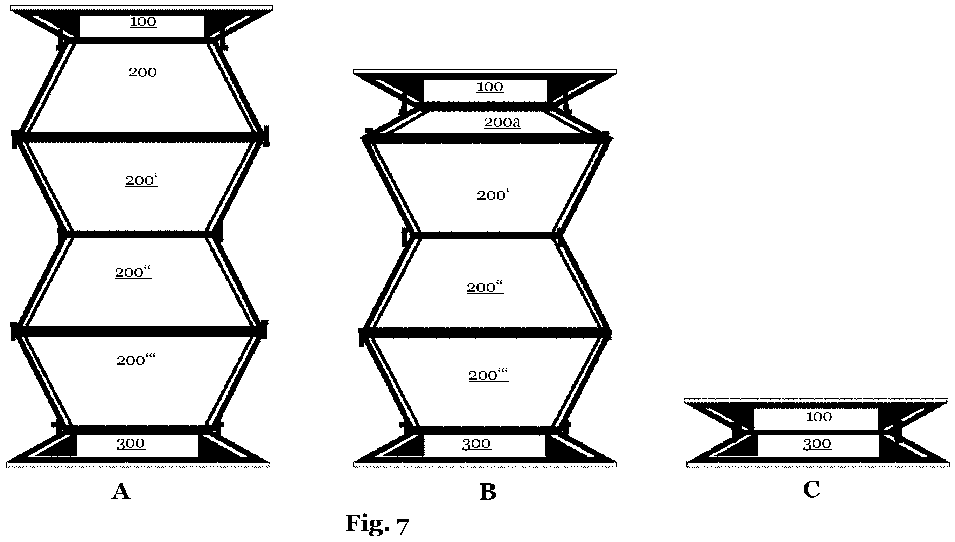

The reservoir elements 100, 200, 200', 300 may form a tank assembly, adapted to be assembled to a tank 1 as shown in Fig. 6. The first ends 112, 212, 312 of each reservoir element have particularly the same size and shape. Likewise, the second ends 114, 214, 314 of each reservoir element have particularly the same size and shape. It is to be understood, as the body elements 110,210, 310 of the respective reservoir elements 100, 200, 300 taper from the first end to the second end, that the size and/or shape of the first and second ends is different. Providing the first ends 112, 212, 312 with particularly the same size and shape and second ends 114, 214, 314 with particularly the same size and shape, allows to assemble the respective reservoir elements of tanks having different volumes (cf. e.g. Fig. 7).

In Fig. 6, an assembled tank 1 for a bio-pharma process line is shown. The tank comprises a top plate element 100, two sidewall elements 200, 200' and a bottom plate element 300. The sidewall elements are the sidewall elements 200, 200' described with respect to Figs. 1 to 3. The bottom plate element and the top plate element may have substantially the same configuration and may be the reservoir elements shown in Figs. 4 and 5, respectively.

The top plate element 100, the sidewall elements 200, 200' and the bottom plate element 300 are arranged to form at least one reservoir 500 for receiving at least one biochemical medium. As the reservoir elements include at least one channel, the tank also comprises at least one channel (not shown), for guiding the at least one biochemical medium and/or an operating medium, wherein the at least one channel extends within at least one of the top plate element, the at least one sidewall element and/or the bottom plate element. At least one channel of each of the reservoir elements 100, 200, 200', 300 may be connected to form a joint channel of the tank.

To form the tank, the second end 114 of reservoir element 100 is sealingly coupled with the second end 214 of reservoir element 100. Likewise, the first ends 112, 112' of reservoir elements 200, 200' are sealingly coupled and the second end 214' of reservoir element 200' is sealingly coupled with the second end 314 of reservoir element 300.

Each of the reservoir elements 100, 200, 300 may include at least one the assembly-connecting means 160, 260, 260' and a corresponding assembly-connecting means 262, 262', 362. The assembly-connecting means can include a latching member, such as a hook, wherein the corresponding assembly-connecting means may include a recess formed for engaging with said hook. Upon engagement, a sealing connection between adjacent reservoir elements can be achieved.

In an alternative embodiment (not shown), the assembly-connecting means may be a threaded member that is integrated into the reservoir element. The threaded member may have an internal thread (e.g. a nut) or an external thread (e.g. a threaded shaft or a screw) and may be integrally formed with the reservoir element. Optionally or additionally, the threaded member may be an inlay, such as a metal inlay, that is securely held in the reservoir element. An inlay can for example be overmolded or glued into the reservoir element. The corresponding assembly-connecting means can be a trough opening that can be aligned with the threaded member having an inner thread. Thus, reservoir elements can be connected and engaged by threading a screw through the through opening into the threaded member. Further, the corresponding assembly-connecting means can be a trough opening that receives a threaded member having an outer thread. Thus, the reservoir elements can be connected and engaged by threading a nut and or the like on the threaded member, thereby engaging the reservoir elements.

Particularly, each of the first and second ends may be associated with an assembly-connecting means and a corresponding assembly-connecting means, so that different reservoir elements can be coupled arbitrary.

Further, the actuating means 152 and 352 of the top plate element 100 and bottom plate element 300 may be connected to each other by respective actuating means 252, 252' of the side wall elements 200, 200'. Thus, e.g. actuating a valve provided in the bottom plate element (not shown) becomes possible by actuating the actuating means 152 of the top plate element 100.

Each of the elements (top plate element 100, sidewall elements 200, 200' and bottom plate element 300) may be adapted to be provided with a sensor and/or a sensor module. A sensor module may comprise multiple sensors, such as at least one of a pH sensor, a temperature sensor, a dissolved oxygen sensor, a biomass sensor, a foam sensor, a pressure sensor, a flow sensor, an O2 sensor, a N2 sensor, a CO2 sensor, and spectroscopy means, such as RAMAN, NIR and/or UV spectroscopy means. The sensor module may be connectable to the respective top plate element 100, sidewall element 200, 200' and/or bottom plate element 300. The sensor module may be provided with a power source such as a rechargeable battery, that allows to operate the sensor module autonomously. Further, the sensor module may comprise a data interface, particularly a wireless data interface for transferring the measured sensor data to a respective control or storing unit.

Further, the tank, particularly the top plate element 100 may comprise at least one a filter. The filter may be provided within the inner volume of the reservoir element 100. Further, the filter may be integrated into the plate element 130 of the reservoir element 100. Even further, an adaptor plate element may be configured to cover a filter and/or a port of the tank at least partially. In a particular embodiment, the top plate element of said tank and an associated adaptor plate element may form a filter housing.

As shown in Fig. 7A to 7C, the reservoir elements are suited to form tanks of different volume. In Fig. 7A, a tank is assembled comprising a top plate element 100, four sidewall elements 200, 200', 200", 200"' and a bottom plate element 300. The sidewall elements are reservoir elements 200, 200', 200", 200'" described with respect to Figs. 1 to 3. The bottom plate element and the top plate element may have substantially the same configuration and maybe the reservoir elements shown in Figs. 4 and 5, respectively. Hence, the tank of Fig. 7A can be assembled from the same elements as the tank shown in Fig. 6, having almost twice the volume.

Fig. 7B shows a further tank, that is similar to the tank shown in Fig. 7A. In this configuration, sidewall element 200 is replaced by a sidewall element 200a. This sidewall element 200a has a smaller height dimension as sidewall element 200, however, first and second ends have particularly the same shape and size as the first and second ends of sidewall element 200. This allows exchanging the sidewall elements 200, 200a.

Fig. 7C shows a configuration having no sidewall elements. The tank is formed from a top plate element 100 and a bottom plate element 300, only. Thus, very little volumes can be achieved.

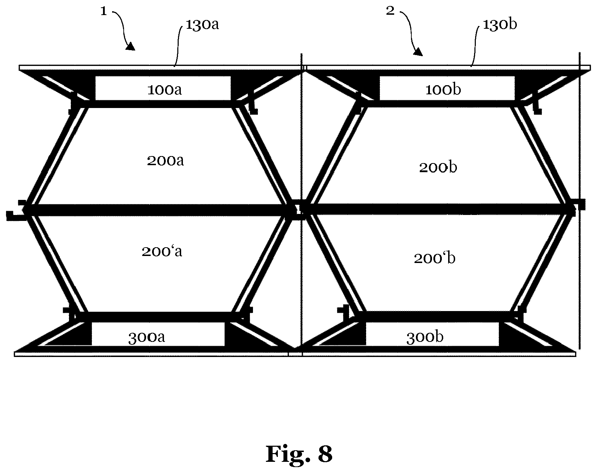

Fig. 8 schematically shows a tank system, comprising two tanks 1,2. Both tanks are configured as described with respect to Fig. 6. The first tank 1 is interconnected with the second tank 2 by at least one connector means (not shown). At least one of the one or more channels of the first tank 1 is fluidically connected to a respective channel of the second tank 2, when the first tank 1 is interconnected with the second tank 2. Hence, medium can be transferred from the first to the second tank, or vice versa.

The top plate elements 100a, 100b, particularly the plate elements 130a, 130b of the first and second tank 1, 2 may protrude laterally over the sidewall elements 200a, 200'a; 200b, 200'b. Hence, connecting the tanks 1, 2 is facilitated, as the sidewall elements do not contravene the connection. Further, the bottom plate elements 300a, 300b may protrude laterally over the sidewall elements 200a, 200'a; 200b, 200'b. Thus, the tanks may be additionally connected via the bottom plate elements.

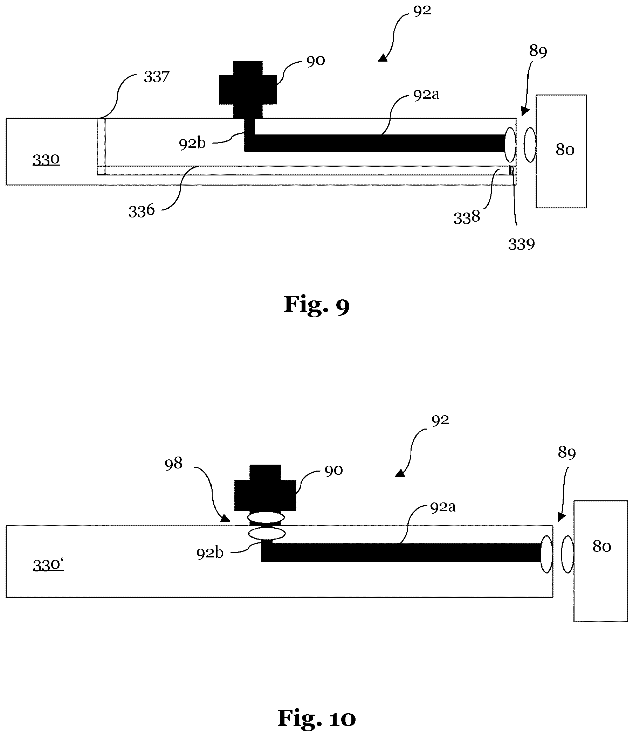

Figs. 9 and 10 schematically show enlarged views of a reservoir element, which may be a bottom plate element 300. Particularly, a plate element, such a plate element 330 of a reservoir element 300 is shown (cf. Fig. 9). Fig. 10 shows a different configuration of a plate element 330'. The plate element 330; 330' includes a stirring means 90, wherein the stirring means may be drivable from the outside of the tank. The stirring means comprises an actuating rod 92, which may include a gearing (not shown). The gearing allows to provide an angle between a drive portion 92a of the actuating rod 92 and an output portion 92b of the actuating rod 92 (here 90 degree). The actuating rod 92 can be coupled with a drive mechanism 80, such as an electric drive mechanism, provided on the outside of the tank. The drive mechanism may be part of a handling manipulator (not shown) that allows automated control of the tank and/or a tank system. The coupling may be achieved by means of a magnetic coupling 89. Thus, when the drive mechanism 80 rotates, the magnetic coupling 89 transfers the rotation to the drive portion 92a. This rotation is then transferred via the output portion 92b to the stirring means 90. The output portion 92b and the stirring means 90 may be magnetically coupled (cf. Fig. 10, magnetic coupling 98) or mechanically coupled, as indicated in Fig. 9.

Further, the plate element 330 may include a channel 336, being associated with two ports 337, 338. Port 337 may be a fluid inlet or outlet port for providing biochemical medium to/from an inner volume of the reservoir element. Port 338 is associated with a valve 339, which may be a magnetic valve. Preferably the magnetic valve can be opened/closed by the drive mechanism 80, inducing a magnetic coupling. In a particular embodiment, the drive mechanism 80 can be moved to the magnetic coupling of the actuating rod 92 and to the magnetic valve 39. Hence, the drive mechanism 80 can be used to operate the valve 339 and the stirring means 90. The magnetic coupling allows to provide the tank in e.g. a clean room bag, while the handling manipulator and the drive 80 are provided outside the clean room bag.

In an alternative embodiment, the drive mechanism may be provided beneath the bottom plate element 300. Thus, no gearing is required.

The magnetic valve 39 may be a needle valve. Said valve may be closed in the initial state and can be opened by axially displacing a valve body. This can e.g. be done by applying a magnetic force. In the closed initial state, the valve may be pre-loaded by means of a spring member or a magnetic member. Hence, when being not actuated, the valve may be in a closed state. Only when being operated (e.g. manually or via a handling manipulator) the valve opens.

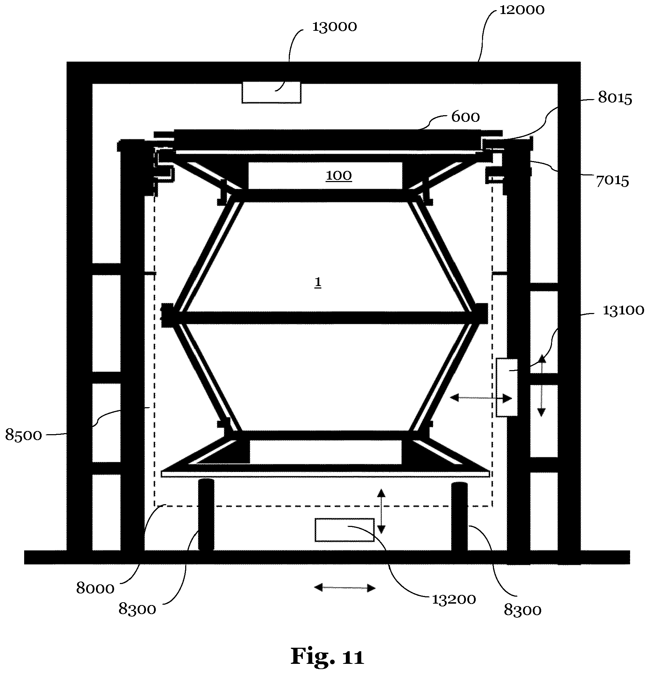

Fig. 11 schematically shows further aspects of a tank system. The tank system comprises multiple tanks, wherein in the perspective of Fig. 11, only a tank 1 is depicted. Said first tan 1 may be interconnected with a second tank (not shown). The tank may be a tank as shown in Fig. 6.

The tank 1 comprises an access plate element, here top plate element 100, that is part of the assembled tank 1. The access plate element 100 is configured to provide access to the tank 1. Further, for improving the guiding of the tanks in the support rail, the top plate elements, sidewall elements and/or the support rail, respectively the support rail sections may include support wheels or support rollers.

The support rollers/wheels may be included in a roller member, that is connectable with a respective top plate element and/or side wall element of the tank. The support rollers/wheels are then guided in the support rail for supporting and guiding the tank in a hanging configuration.

The system may further comprise at least one handling manipulator 13000, 13100, 13200. For example, a first handling manipulator 13000 may be arranged above the tank 1, a second handling manipulator 13100 may be arranged below the tank 1, and a third handling manipulator 13200 may be arranged besides the tank 1. The handling manipulator(s) are arranged movably with respect to the tank 1 so as to access and to control at least one tank e.g. via the access plate element 100. Particularly, the handling manipulator may include guide rails 12000, that allow moving the handling manipulator in at least one axial direction. In a particular embodiment, the handling manipulator includes a gantry robot.

Particularly, the handling manipulators 13000, 13100, 13200 may be adapted to provide an operating medium to the tank 1 and/or to removing operating medium from the respective tank, so as to transfer the biochemical medium to and/or out of the tank.

The handling manipulators 13000, 13100, 13200 may comprise driving means (not shown) that can couple with an actuating means of the tank for driving a pump, a string means and/or the like and/or for opening/closing valves.

Further, the handling manipulators 13000, 13100, 13200 may be configured to supply/retain biochemical and/or operating medium to the tank(s). Particularly, an operating medium, such as pressurized air, may be used to transfer a biochemical medium to and/or out of the tank. Therefore, the tank is operable via the operating medium. For transferring a biochemical medium out of the tank, a positive pressure can be applied (e.g. by the handling manipulator 13000, 13100, 13200) to the first tank 1, by applying operating medium to the tank. Thus, biochemical medium is urged out of the tank 1, e.g. towards a second tank 2 and/or a filter.

For transferring biochemical medium into the tank, a negative pressure can be established, particularly by the handling manipulator 13000, 13100, 13200, e.g. by removing operating medium from the tank. In case pressurized air is used as operating medium, preferably sterile pressurized air is used. Sterile pressurized air can be obtained by guiding pressurized air through a respective sterile filter prior to entering the tank.

An operating medium, such as pressurized air can be supplied to or removed from the tank 1 by a respective gas inlet and/or outlet port using the handling manipulator 13000, 13100, 13200. The application and/or removal of medium from the tank may for example be performed pressure controlled, volume controlled, and/or mass controlled.

Further, at least one handling manipulator, such as handling manipulator 13200, may include a weighing member, allowing to weigh a tank of the tank system. The weight of the respective tanks may then be used to control a process line formed by the tanks.

The system may further include at least one clean room bag 8000 (indicated as dashed line in Fig. 11). Clean room bag 8000 comprises at least one foil portion 8500. The clean room bag is configured to receive at least one tank 1 and to provide a clean room environment for the at least one tank 1 when the at least one tank 1 is received in the clean room bag 8000. The at least one tank 1 is accessible with a handling manipulator 13000, 13100, 13200 from the outside of the clean room bag 8000.

Further, the system includes at least one adaptor plate element 600. The adaptor plate element 600 is associated with at least one tank 1 and can be installed on the at least one tank, so that one of the at least one foil portions 8500 of the clean room bag 8000 is sandwiched between an access plate element 100 of the tank 1 and the adaptor plate element 600.

The adaptor plate element 600 is configured to define at least one access point (not shown) to provide access to the tank from the outside of the clean room bag 8000, via the access plate element 100. For example, the adaptor plate element 600 is configured to cover a filter and/or a port of the tank 1 at least partially.

The foil portion 8500 is provided removably and/or pierceable in an area of the filter and/or port of the tank to provide access to the tank 1. The adaptor plate element 600 may further be configured to support an actuating means 152 of a valve (not shown).

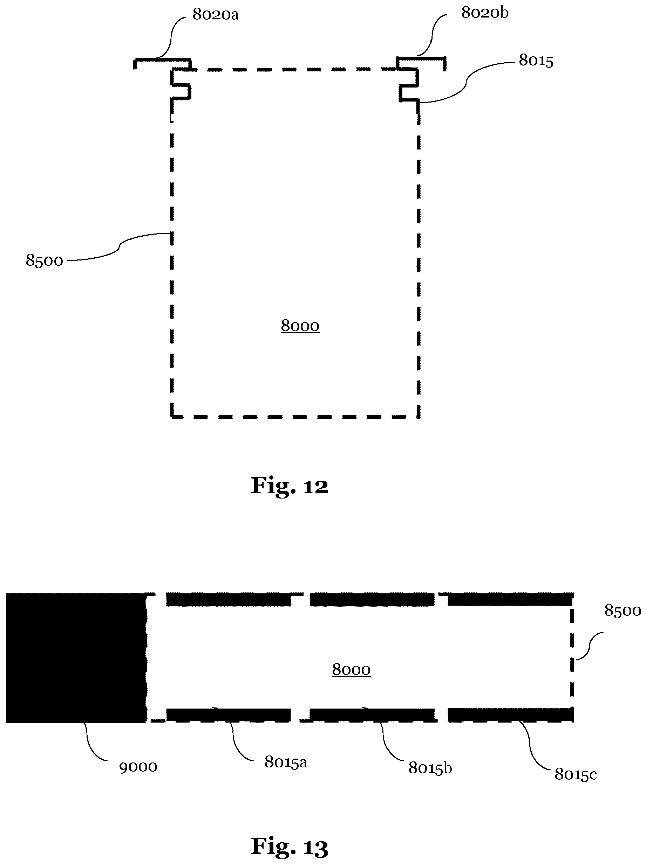

As best shown in Fig. 12, the clean room bag 8000 comprises at least one support rail 8015 for guiding at least one received assembled tank within the clean room bag 8000. The support rail 8015 being adapted to support at least two tanks, wherein the support rail 8015 engages with the top plate elements 100 of the respective tanks 1, so that the top plate elements 100 of tanks with different height dimensions are arranged on the same level, when being supported by the support rail 8015.

Further, as shown in Fig. 13, the support rail 8015 maybe formed from multiple support rail sections 8015a, 8015b, 8015c. These sections may be separated by foil portions, so that the clean room bag 8000 is foldable. Particularly, the support rail sections 8015a, 8015b, 8015c may be made from a plastic material and may be injection moulded. In a particular embodiment, the foil portion 8500 and the support rail sections 8015a, 8015b, 8015c are fixedly adhered to each other (e.g. welded or glued) or even integrally formed. To enhance the load capacity of the support rail sections 8015a, 8015b, 8015c, those sections may include a metallic insert.

Further, the support rail sections 8015a, 8015b, 8015c can be configured to receive a corresponding support rail of a frame 7000, which may be part of the handling manipulator. The frame 7000 may include a rigid corresponding support rail 7015, such as a metallic support rail. The support rail sections 8015a, 8015b, 8015c can be formed so as to fit the corresponding support rail 7015 of the frame 7000. Further, the support rail sections 8015a, 8015b, 8015c may include a latching means 8020a, 8020b, that allow to engage the support rail sections 8015a, 8015b, 8015c with the corresponding support rail 7015 of the frame 7000, preferably by snap fit.

The support rail may include a weighing member, allowing to weigh the supported tanks individually. The weight of the respective tanks may then be used to control a process line formed by the tanks.

As shown in Fig. 11 Further, the support rail 8015 supports the tank 1 in a hanging configuration, wherein the bottom plate element 300 has no contact to the ground. Additionally, support members 8300 may be provided that support that tank 1 at the bottom plate element 300 additionally to the hanging support of the top plate element 100. Said support members may be adapted to be adjustable in height, e.g. by a linear drive, such as a magnetic drive, an electric drive, a pneumatic drive and/or a hydraulic drive. The support members may include a weighing member, allowing to weigh the supported tank. The weight of the respective tank may then be used to control a process line.

The clean room bag 8000 may further include a sealable opening (not shown) that is configured for receiving assembled tanks, wherein the sealable opening of the clean room bag is configured to open jointly with a corresponding sealable opening of an assembly room 9000, when the clean room bag is sealingly coupled to the assembly room. Further, a sealable opening may be used to remove tanks from the clean room bag. The clean room bag may include multiple sealable openings. Thus, transferring assembled tanks from the assembly room to the clean room bag or out of the clean room bag is facilitated.

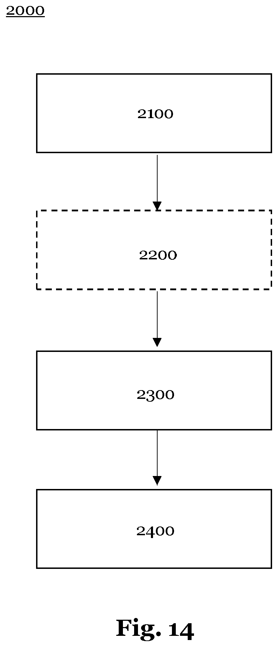

Fig. 14 schematically shows a flow diagram of a method 2000 for assembling a tank. The method comprises the following steps: providing 2100 a top plate element; optionally providing 2200 at least one sidewall element; providing 2300 a bottom plate element and assembling 2400 the top plate element and the bottom plate element and optionally the sidewall element(s) to form a reservoir for receiving at least one biochemical medium.

## Claims

1. A reservoir element (200) for a tank (1) of a bio-pharma process line, the reservoir element (200) including
a body element (210), and
at least one shell element (220, 222),
the body element (210) having a first end (212) and a second end (214), being opposite the first end, each of the first and second ends including an orifice, and wherein the body element tapers from the first end to the second end, wherein
an inner volume is defined between the first and second ends, and wherein
the body element and/or the at least one shell element include at least one groove (216, 217), wherein
the at least one shell element (220, 222) is fixedly attached to the body element (210) so that the body element and the at least one shell element sandwich the groove in between forming at least one channel,
the at least one channel being adapted for guiding at least one biochemical medium and/or an operating medium.

2. The reservoir element (200) of claim 1, wherein the body element has a polygonal cross-section in a plane parallel to the first end (212), and wherein the at least one shell element (220, 222) may be plate shaped.

3. The reservoir element (200) of any preceding claim, wherein the inner volume and the first orifice are configured so that the reservoir element (200) is adapted to receive a further reservoir element (200') at least partially, so that multiple reservoir elements are stackable within each other.

4. The reservoir element (100; 300) of any of any preceding claim, further comprising a plate element (130; 330),
the plate element (130; 330) being sealingly attached to the first end (112; 312) or second end (114; 314) of the body element, so as to cover the respective orifice, wherein
the plate element may include at least one channel, wherein
the at least one channel is in communication with the channel formed by the body element and the at least one shell element.

5. The reservoir element (100; 300) of any preceding claim, further comprising an insert (140; 340), adapted to cover an undercut of the inner volume at least partially.

6. The reservoir element (100; 200; 300) of any preceding claim, wherein the first end and/or second end include a sealing face (215), for sealingly connect the reservoir element to a further reservoir element.

7. The reservoir element (100; 200; 300) of any preceding claim, wherein the reservoir element (100; 200; 300), particularly the plate element, includes at least one of the following:
• at least one port (230; 337, 338), wherein the at least one port is associated with a respective channel (336), and wherein the port is chosen from a group of port-types, comprising the following port-types:
∘ a fluid inlet port;
∘ a gas inlet port;
∘ a fluid outlet port;
∘ a heating/cooling port;
∘ a gas outlet port;
∘ a cell bleed port,
∘ a cell transfer port,
∘ a medium supply port,
∘ a medium remove port,
∘ an element-interconnecting port, and
∘ a tank-interconnecting port;
• at least one filter, wherein the at least one port (230) may be covered by the at least one filter, and wherein the filter may be chosen from a group of filter-types, comprising the following filter-types:
∘ a pre-filter;
∘ a sterile filter;
∘ a bacterial filter;
∘ a viral filter;
∘ a mycoplasma filter;
∘ an ultrafiltration filter;
∘ a diafiltration filter;
∘ a cell filter;
∘ a cell harvest filter;
∘ a fluid filter;
∘ a bioburden filter;
∘ an air filter, and
∘ a gas filter, wherein
the filter covering the at least one port may be heated and/or cooled;
• at least one valve (339), the at least one valve being associated with the at least one channel (336), wherein the valve may be a flow control valve, a cutoff valve, a pressure relief valve or a non-return valve, and wherein the valve may be a mechanical or a magnetic valve that is configured to be actuatable from the outside of the tank, by means of an actuating means (152).

8. The reservoir element (100; 200; 300) of any preceding claim, comprising at least one assembly-connecting means (160, 260, 260') and/or at least one corresponding assembly-connecting means (262, 262', 362), wherein
the assembly-connecting means (160, 260, 260') and the corresponding assembly-connecting means (262, 262', 362) are configured to engage with each other, so as to secure an assembly of at least two adjacent reservoir elements, wherein the engagement of the assembly-connecting means (262, 262', 362) and the corresponding assembly-connecting means (262, 262', 362).

9. A tank assembly adapted to be assembled to a tank (1), wherein the tank assembly comprises
at least two reservoir elements (100; 300) according to any one of claims 4 to 8, and wherein
the tank assembly particularly includes two reservoir elements (100; 300) according to any one of claims 4 to 8 and at least two further reservoir elements (200) according to any one of claims 1 to 8, wherein
the first ends of each reservoir element have particularly the same size and shape and wherein the second ends of each reservoir element have particularly the same size and shape.

10. A tank (1) for a bio-pharma process line, the tank comprising:
a top plate element (100), optionally at least one sidewall element (200, 200', 200", 200"'), and a bottom plate element (300), wherein
the at least one sidewall element is a reservoir element according to any one of claims 1 to 3 or 5 to 8, and wherein the
top plate element (100) and the bottom plate element (300) are reservoir elements according to any one of claims 4 to 8, wherein
the top plate element, optionally the at least one sidewall element and the bottom plate element are arranged to form at least one reservoir (500) for receiving at least one biochemical medium, and wherein
the tank (1) comprises further
at least one channel, for guiding the at least one biochemical medium and/or an operating medium, wherein the at least one channel extends within at least one of the top plate element, the at least one sidewall element and/or the bottom plate element, wherein the length of the at least one channel is longer than the thickness of the respective top plate element, sidewall element and/or the bottom plate element.

11. A tank system, comprising multiple tanks (1, 2), according to claim 10, wherein
a first tank (1) is interconnectable with a second tank (2) by at least one connector means, when the second tank (2) is arranged directly adjacent to the first tank (1), and wherein
at least one of the one or more channels of the first tank is fluidically connected to a respective channel of the second tank (2), when the first tank (1) is interconnected with the second tank (2).

12. The tank system according to claim 11, further comprising a support rail (7015, 8015), the support rail being adapted to support at least two tanks (1, 2), wherein the support rail engages with the top plate elements (100) and/or sidewall elements (200) of the respective tanks, so that top plate elements of tanks with different height dimensions are arranged on the same level, when the tanks are supported by the support rail.

13. The tank system according to any one of claims 11 or 12, further comprising
a clean room bag (8000) that comprises at least one foil portion (8500), wherein the clean room bag is configured to receive at least one tank (1, 2) and to provide a clean room environment for the at least one tank when the at least one tank is received in the clean room bag, wherein
the at least one tank is accessible with a handling manipulator from the outside of the clean room bag,
the system optionally further comprising
at least one adaptor plate element (600), wherein the adaptor plate element is associated with at least one tank and can be installed on the at least one tank, so that one of the at least one foil portions (8500) of the clean room bag is sandwiched between an access plate element (100) of the tank and the adaptor plate element (600),
wherein at least one of the top plate element, the at least one sidewall element and the bottom plate element is said access plate element, wherein
the adaptor plate element (600) is configured to define at least one access point to provide access to the tank from the outside of the clean room bag, via the access plate element, wherein further optionally
the adaptor plate element (600) is configured to cover a filter and/or a port of the tank at least partially, and wherein the foil portion (8500) is provided removably and/or pierceably in an area of the filter and/or port of the tank to provide access to the tank and/or, wherein the adaptor plate element (600) is configured to support an actuating means of a valve and/or an operating means of the tank, and wherein the foil portion is provided removably and/or pierceably in an area of the actuating means of a valve and/or an operating means of the tank to provide access to the tank.

14. A clean room bag (8000), for being used in system according to any one of the previous system claims,
the clean room bag (8000) being configured to receive at least one tank (1, 2) and to provide a clean room environment for the at least one tank when the at least one tank is received in the clean room bag, wherein
the clean room bag comprises at least one foil portion (8500), that is adapted to be sandwiched between an access plate element (100) of the tank and an adaptor plate element (600), so as to define at least one access point to provide access to the tank from the outside of the clean room bag, via the access plate element, the clean room bag further comprising at least one support rail (8015) for guiding at least one received assembled tank within the clean room bag,
the support rail being adapted to support at least two tanks, wherein the support rail may engage with the top plate elements (100) and/or the sidewall elements (200) of the respective tanks, so that the top plate elements of tanks with different height dimensions are arranged on the same level, when the tanks are supported by the support rail,
wherein the support rail may be formed from multiple support rail sections (8015a, 8015b, 8015c), so that the clean room bag is foldable.

15. A method (2000) for assembling a tank (1) according to claim 10, wherein the method comprises the following steps:
providing (2100) a top plate element;
optionally providing (2200) at least one sidewall element;
providing (2300) a bottom plate element;
assembling (2400) the top plate element, the bottom plate element and optionally the at least one sidewall element, to form a reservoir for receiving at least one biochemical medium.
